# EUROPEAN PATENT SPECIFICATION

(11) **EP 2 421 507 B1**
(45) Date of publication and mention of the grant of the patent: **23.01.2019**
(21) Application number: 10716463.4
(22) Date of filing: 26.04.2010
(51) Int. Cl.: A61K 9/14, A61K 9/68, A61K 31/465

(54) **CHEWING GUM AND PARTICULATE MATERIAL FOR CONTROLLED RELEASE OF ACTIVE INGREDIENTS**
KAUGUMMI UND TEILCHENFÖRMIGES MATERIAL ZUR KONTROLLIERTEN FREISETZUNG VON WIRKSTOFFEN
GOMME À MÂCHER ET MATIÈRE PARTICULAIRE POUR LA LIBÉRATION CONTRÔLÉE DE PRINCIPES ACTIFS

(30) Priority: 24.04.2009 US 172474 P
(43) Date of publication of application: 29.02.2012
(73) Proprietor: Fertin Pharma A/S, 7100 Vejle (DK)
(72) Inventor: PEDERSEN, Kurt, Møller, DK-7323 Give (DK); ANDERSEN, Jette, Baek, DK-7100 Vejle (DK)
(74) Representative: Patentgruppen A/S
(86) International application number: PCT/DK2010/000055
(87) International publication number: WO 2010/121619

(56) References cited:
- EP-A1- 1 974 807
- EP-A1- 2 168 572
- EP-A2- 0 219 458
- WO-A2-2005/023227
- WO-A2-2005/072125
- GB-A- 1 572 718
- US-A- 1 171 392
- US-B1- 6 344 222

## Description

### FIELD OF THE INVENTION

The present invention relates to the field of chewing gum comprising a particulate material for controlled release of nicotine. In particular, the present invention relates to a chewing gum comprising inorganic mineral filler, such as a natural calcium carbonate or a precipitated calcium carbonate. The particulate material is suitable for controlled relase of active ingredients in various products, such as chewing gum products.

### BACKGROUND OF THE INVENTION

It is a general understanding that controlled release of active ingredients may be a critical parameter in terms of the ability to deliver an active ingredient in the most suitable manner. This applies to confectionery products where a controlled release of active ingredients, such as nutraceuticals or oral care agents, is critical to give the desired effect. This applies also to pharmaceutical products where active pharmaceutical ingredients may be harmfull or even lethal in a high dose. In addition it may be beneficial that the release of active ingredients is controlled in order to achieve an improved experience of the product, such as a better taste of the product or a masking effect.

Although various solutions have been provided in the past, the solutions imply several drawbacks, which have been difficult or sometimes impossible to solve.

WO 2005/072125 discloses a composite comprising a basic inorganic material, a water-soluble compound adsorbed to said basic inorganic material, and a biodegradable polymer which yields acidic degradation products.

GB 1 572 718 discloses a method for reducing the amount of biologically active substance required for obtaining a predetermined biological effect.

EP 1 974 807 discloses a process for the removal of endocrine disrupting compounds (EDCs) from an aqueous medium, wherein a surface-reacted natural calcium carbonate or an aqueous suspension comprising a surfacereacted calcium carbonate is brought into contact with the EDC containing medium.

EP 0 219 458 discloses a carrier for the controlled release of active agents, comprising surface reacted natural or synthetic calcium carbonate, one or more active agents, wherein said one or more active agent is associated with said surface-reacted calcium carbonate.

US 6 344 222 discloses that certain filler materials may enhance the release and absorption of nicotine and other tobacco alkaloids in chewing gum compositions. Desirable filler materials include calcium carbonate, magnesium silicate (talc), as well as dicalcium phosphate, and any mixtures thereof.

### SUMMARY OF THE INVENTION

The present inventors have surprisingly found that inorganic mineral filler, such as natural calcium carbonate, is a highly suitable carrier of nicotine, without the drawbacks of the prior art.

The inorganic mineral filler of the present invention provides a surprisingly high loading capacity of active ingredients, such as nicotine. The high loading capacity may be a benefit in a number of applications. One advantage is that a higher content of active ingredients may be applied. Another advantage is that active ingredients may be effectively separated from other ingredients in the formulation, whereby unfavorably interaction between the ingredients may be avoided. Yet another advantage is that a high loading capacity implies a better stability of active ingredient.

In addition, the active ingredient may be reversibly absorbed into and/or adsorbed onto the inorganic mineral filler, which is highly advantageous in order for a controlled release of active ingredients.

The particulate material of the present invention may give an immediate release of nicotine or may be designed to sustain the release for longer periods. In particular the present invention may provide sustained release of nicotine in a much better way compared to the prior art. The porous nature of inorganic mineral filler, such as natural calcium carbonate, offers a much better stability of the active ingredients, and is tasteless, stabile and biocompatible. It exhibits good compactability in drug mixtures.

Compared to other carrier materials, natural calcium carbonate may in particular be useful. The present invention is particularly suitable for delivery of nicotine and the present invention is suitable for controlled release in chewing gum.

In particular when chewing gum is used as the delievery dehicle, the particulate material of the present invention may be incorporated in the core or in a sub-layer between the chewing gum core and an outer coating.

Accordingly, there is provided a particulate material for controlled release of active ingredients, the particulate material comprising a combination of one or more active ingredients, including nicotine, and an inorganic mineral filler, wherein the active ingredient is reversibly absorbed into and/or adsorbed onto the inorganic mineral filler, and wherein the BET specific surface area of the inorganic mineral filler is above 15 m²/g, the BET specific surface area measured in accordance with ISO 9277.

Furthermore, the present invention provides a chewing gum having controlled release of one or more active ingredients including nicotine, comprising a chewing gum core and a particulate material.

Finally, the present invention provides a method of manufacture a chewing gum comprising the steps of:
i) preparing a combination of an active ingredient being nicotine and an inorganic mineral filler, wherein the active ingredient is reversibly absorbed into and/or adsorbed onto the inorganic mineral filler, obtaining a particulate material, wherein the particulate material comprises a combination of one or more active ingredients, such as nicotine or a flavoring agent, and an inorganic mineral filler, and wherein the BET specific surface area of the inorganic mineral filler is above 15 m²/g, the BET specific surface area measured in accordance with ISO 9277,
ii) formulating the particulate material with chewing gum ingredients, including gum base, obtaining a chewing gum having controlled release of the active ingredient, and
iii) optionally, pre-treating the inorganic mineral filler prior to step i) with one or more medium-strog to strong providers of H₃O⁺ ions and/or pre-treated with an inorganic salt, such as a magnesium sulphate in combination with aluminium sulphate and/or zinc sulphate, and pre-treated with gaseous CO₂, and for precipitated calcium carbonate preferably at a CO₂ gas flow rate of below 30 litres per minute at standard temperature and pressure per kilogram calcium hydroxide during precipitation.

### DETAILED DESCRIPTION

The present invention provides for a particulate material, a chewing gum and a method, where an active ingredient being nicotine is absorbed into and/or adsorped onto an inorganic mineral filler, such as natural calcium carbonate. Thereby nicotine is stabilised. The release of the nicotine from the nicotine-carbonate mixture may be immediate or may be controlled so that the nicotine is delivered at a pre-determed time.

Furthermore, the present invention provides for different dosage forms such as a chewing gum, and compressed chewing gum.

The term "compressed chewing gum" is used in this document to indicate a chewing gum manufactured by compressing granules and optionally other ingredients at a certain pressure to obtain a chewing gum. The term "tabletting" used in this document is synonymous with compressing, whereas the term tabletting in prior art sometimes indicate the process of making standard chewing gum pieces (tablets) by punching or the like.

According to the present invention, the particulate material according to the present invention is chemically and physically stable. In the present context the term "stable" means that the inorganic mineral filler combined with the active ingredient is chemically and/or physically stable for at least about 22 weeks such as, e.g., at least 14 weeks when stored open at a temperature of 40 °C and a relative humidity of 50%.

It is especially of importance that the nicotine does not migrate out of the inorganic mineral filler as such a migration will lead to a marked loss in the content of active ingredient in the material. Furthermore, nicotine when used in the present invention as a base is a volatile substance and, therefore, it is normally difficult to maintain a relatively constant concentration of the nicotine base in a composition comprising a mere admixture of nicotine and a pharmaceutically acceptable excipient. A particulate material according to the present invention is also physically stable. Thus, within a time period of 22 weeks or more no visible changes had been observed and the dissolution profile did not change.

With reference to this invention, the term "chewing gum" means all chewable gum products. The term "API" intends to mean active pharmaceutical ingredient. The term "nicotine" intends to mean nicotine in any form, be it the free base form, a salt, a complex or any other form.

The terms "buccal" and "buccally" and equivalents are herein intended to pertain to all of or any part of the tissue of the oral cavity.

The term "surface area" and equivalents are deemed according to the following method: The specific surface area of the powders was obtained from a BET analysis of N2 adsorption isotherms (ASAP 2010, Micromekics, USA). From the same set of measurements, the total pore volume of the powders was obtained by the ASAP 2010 V4 software. The weight of the samples in these measurements was chosen so as to produce a total surface of 5-10 m2.

The present invention concerns a chewing gum comprising a particulate material for controlled release of nicotine , the particulate material comprising a combination of one more active ingredients, including nicotine, and an inorganic mineral filler, wherein the nicotine is reversibly absorbed into and/or adsorbed onto the inorganic mineral filler, and wherein the BET specific surface area of the inorganic mineral filler is above 15 m²/g, the BET specific surface area measured in accordance with ISO 9277..

In order to obtain a suitable effect when nicotine is used as the active ingredient, a particulate material according to the present invention is a material, which - when administered to at least one healthy subject in the form of a chewing gum containing a particulate material containing 4 mg nicotine or an equivalent amount of a pharmaceutically acceptable salt, complex or solvate thereof - results in a plasma concentration of at least about 5 ng/ml such as, e.g., about 6 ng/ml or more, about 7 ng/ml or more, about 8 ng/ml or more, about 9 ng/ml or more or about 10 ng/ml or more of nicotine within at the most about 30 min such as, e.g. within at the most about 25 min, within at the most about 20 min, within at the most about 15 min or within at the most about 10 after administration.

In certain cases a very fast onset of action is desired when using nicotine as the active ingredient, and the nicotine contained in a particulate material or a composition according to the invention must therefore be released relatively fast. In such specific embodiments a particulate material according to the present invention is a material, which - when administered to at least one healthy subject in the form of a chewing gum containing a particulate material containing 4 mg nicotine or an equivalent amount of a pharmaceutically acceptable salt, complex or solvate thereof - results in a plasma concentration of at least about 5 ng/ml such as, e.g., about 6 ng/ml or more, about 7 ng/ml or more, about 8 ng/ml or more, about 9 ng/ml or more or about 10 ng/ml nicotine within at the most about 15 min such as, e.g. within at the most about 10 min, within at the most about 7.5 min, within at the most about 5 min, within at the most about 4 min or within at the most about 3 min after administration.

The absorption rate when nicotine is used as the active ingredient is controlled primarily by the release rate from the carrier and pH (amount uncharged nicotine) at the absorptive site. In order to adjust pH at the absorption site, one or more buffer substances may be incorporated into the particulate material or composition of the present invention. The buffer substance may at least partially be sorbed by the inorganic mineral filler. Suitable buffer substances for use in the present context are e.g. alkaline or alkaline earth metal salts such as, e.g., sodium hydroxide, potassium hydroxide, magnesium hydroxyde, carbonates including monocarbonate, bicarbonate and sesquicarbonate such as, e.g, sodium carbonate, sodium hydrogen carbonate, potassium carbonate, potassium hydrogen carbonate, magnesium carbonate, calcium carbonate etc., glycinate, phosphate including monohydrogenphosphate, dihydrogenphosphate and trihydrogenphosphate, glycerophosphate, gluconate, borate, ammonium, and mixtures thereof salts of organic or inorganic acids such as, e.g., acetates, citrates, tartrates etc.,

Nicotine according to the present invention is nicotine selected from nicotine salts, preferably tartrate, hydrogen tartrate, hydrochloride, acetate or salicylate, the free base form of nicotine, a nicotine derivative, nicotine in any non-covalent binding, and mixtures thereof.

According to the present invention, nicotine gum is found in two strengths, 2 and 4 mg, which is the actual content of nicotine. However the biological available dose from these gums are only about 1 and 2 mg, respectively. This is because it is difficult to completely empty the gum and some of the nicotine that is extracted from the gum cannot be absorbed into the circulatory system due to swallowing into the gastrointestinal tract where most of the nicotine is metabolised before entering the circulation. Moreover the speed by which the nicotine is absorbed is much slower than from the cigarette. This is an important factor since when individuals want to change their state of mind with psychoactive drugs the effects are strongly dependent on the speed by which the drug enters the brain.

According to the present invention a further active ingredient may be absorbed into or adsorbed onto the particulate material. Such further active ingredients include active pharmaceutical ingredients (API), such as nicotine. Other suitable APIs are preferably selected among the below listed compounds.

Teeth whitening actives may be included in the present invention. The actives suitable for whitening are selected from the group consisting of oxalates, peroxides, metal chlorites, perforates, percarbonates, peroxyacids, and mixtures thereof. Suitable peroxide compounds include hydrogen peroxide, calcium peroxide, sodium peroxide, carbamide peroxide, urea peroxide, sodium percarbonate and mixtures thereof. Optionally, the peroxide is hydrogen peroxide. Suitable metal chiorites include calcium chlorite, barium chlorite, magnesium chlorite, lithium chlorite, sodium chlorite and potassium chlorite. Additional whitening actives may be hypochiorite and chlorine dioxide. A preferred chlorite is sodium chlorite. The effectiveness of whitening actives can, optionally, be enhanced by means of a catalyst, i.e. a two- component peroxide-catalyst system. Useful whitening agent catalysts or catalytic agents can be found in U.S. Patent Number 6,440,396 to McLaughlin, herein incorporated by reference in its entirety as to the description of whitening agents and systems.

When incorporating peroxide actives, the particulate material of present invention can, optionally, contain peroxide active stabilizers. Peroxide active stabilizers suitable for use herein include, but are not limited to, polyethylene glycols such as PEG 40 or PEG 600; zinc salts such as zinc citrate; polyoxyalkylene block-polymers (e.g. Pluronics); aminocarboxylic acids or salts thereof; glycerols; dyes such as Blue #1 or Green #3; phos- phates such as phosphoric acid, sodium phosphate or sodium acid pyrophosphate; stannous salts such as stannous chloride; sodium stannate; citric acid; etidronic acid; carbomers or carboxypolymethylenes such as those of the Carbopol® series, butylated hydroxytoluene (BHT), ethylenediaminetetraacetic acid (EDTA) and mixtures thereof.

Anti-tartar agents useful herein include phosphates. Phosphates include pyrophosphates, polyphosphates, polyphosphonates and mixtures thereof. Pyrophosphates are among the best known phosphates for use in dental care products. Pyrophosphate ions delivered to the teeth derive from pyrophosphate salts. The pyrophosphate salts useful in the present compositions include the dialkali metal pyrophosphate salts, tetra-alkali metal pyrophosphate salts, and mixtures thereof. Disodium dihydrogen pyrophosphate (Na2H2P2O7), tetrasodium pyrophosphate (Na4P2O7), and tetrapotassium pyrophosphate (K4P207) in their non-hydrated as well as hydrated forms are preferred. Anticalculus phosphates include potassium and sodium pyrophosphates; sodium tripolyphosphate; diphosphonates, such as ethane-1-hydroxy-1,I-diphosphonate; 1 -azacycloheptane- 1,1 - diphosphonate; and linear alkyl diphosphonates; linear carboxylic acids and sodium and zinc citrate.

Agents that may be used in place of or in combination with the above pyrophosphate salt include materials such as synthetic anionic polymers including polyacrylates and copolymers of maleic anhydride or acid and methyl vinyl ether, e.g. Gantrez, as described, for example, in U. S. Patent Number 4,627,977, to Gaffar et al. herein incorporated by reference in its entirety as to the description of such agents, as well as e.g. polyamino propane sulfonic acid (AMPS), zinc citrate trihydrate, polyphosphates, e.g. tripolyphosphate and hexametaphosphate, diphosphonates, e.g. EHDP and AMP, polypeptides, such as polyaspartic and polyglutamic acids, and mixtures thereof.

Antimicrobial agents can also be present in the particulate material of the present invention as oral agents and/or systemic actives. Such agents may include, but are not urn- ited to, 5-chloro-2-(2, 4-dichlorophenoxy)- phenol, commonly referred to as triclosan, chiorhexidine, alexidine, hexetidine, sanguinarine, benzalkonium chloride, salicylamide, domiphen bromide, cetylpyridiurn chloride (CPC), tetradecyl pyridinium chloride (TPC); N-tetradecyl-4- ethyl pyridinium chloride (TDEPC); octenidine; delmopinol, octapinol, and other piperidino derivatives, niacin preparations; zinc/stannous ion agents; antibiotics such as AUGMENTIN, amoxycillin, tetracycline, doxycyline, minocycline, and metronidazole; and analogs, derivatives and salts of the above antimicrobial agents and mixtures thereof.

Anti-inflammatory agents can also be present in the particulate material of the present invention as oral agents and/or systemic actives. Such agents may include, but are not limited to, non- steroidal anti- inflammatory agents or NSAIDs, such as propionic acid derivatives; acetic acid derivatives; fenamic acid derivatives; biphenylcarboxylic acid derivatives; and oxicams. All of these NSAIDs are fully described in U.S. Patent Number 4,985,459 to Sunshine et al., incorporated by reference herein in its entirety as to the description of such NSAJDs. Examples of useful NSAIDs include acetylsalicylic acid, ibuprofen, naproxen, benoxaprofen, flurbiprofen, fenoprofen, fenbufen, ketoprofen, indoprofen, pirprofen, carprofen, oxaprozin, pranoprofen, microprofen, tioxaprofen, suprofen, alminoprofen, tiaprofenic acid, fluprofen, bucloxic acid and mixtures thereof.

Also useful are the steroidal anti-inflammatory drugs such as hydrocortisone and the like, and COX-2 inhibitors such as meloxicam, celecoxib, rofecoxib, valdecoxib, etoricoxib or mixtures thereof. Mixtures of any of the above anti-inflammatories may be used.

Other materials that can be used with the present invention include commonly known mouth and throat products. These products include, but are not limited to, upper respiratory agents such as phenylephrine, diphenhydramine, dextromethorphan, bromhexine and chiorpheniramine, gastrointestinal agents such as famotidine, loperamide and sirnethicone, anti-fungals such as miconazole nitrate, antibiotics and analgesics such as ketoprofen and fluribuprofen.

The particulate material may comprise a flavoring agent in a chewing gum formulation. One particular embodiment comprises embedding in a sub-layer between the chewing gum core and an outer coating to prolong the flavoring sensation.

In order to reduce manufacturing costs and in order to facilitate product approval for similar chewing gums by health authorities it is often desirable to use the same core with differently flavored coatings. Thereby the flavor of the core needs to be dominated by the flavor of the coating(s). This effect is obtained by the present invention where the flavor of the at least the sub-layer may dominate over the flavor of the core.
An example of such domination is when a fruit flavor in the sub-layer dominates over a mint flavor of the core. The mechanism behind this flavor domination is that the flavor in the polymer coating has a slow release therefrom. Further, upon chewing, part of the sub-layer gets embedded in the core, from where the sub-layer flavor is subsequently slowly released. Sub-layers also allows for addition of a large percentage of flavor as compared to a hard coating.

For flavored nicotine-containing chewing gums the invention provides a solution to the combined problem of obtaining a long lasting effect of the flavoring agent(s), obtaining domination of flavoring agents in the coating(s) over flavoring agent(s) in the core, avoiding problems of chemical or pharmaceutical incompatibility between nicotine in the core and flavoring agent(s) in the coating(s), and/or increasing the control of the release of the drug. Known techniques for flavoring chewing gums imply that flavoring agents are added to a gum core and optionally to a hard coating on the core. Anyhow, such flavoring does not solve the preceding problem.

According to the present invention said combined problem may be solved by providing a chewing gum core with at least one sub-layer, whereby the flavoring agent(s) is/are added to at least the sub-layer. The nicotine may be in the core and/or in one or more of the coatings. For a nicotine-containing chewing gum the nicotine is preferably in the core and in an at least one sub-layer for providing quick nicotine release from the sub-layer and slow nicotine release from the core.

According to one aspect of the invention, the chewing gum, when used, may comprise nicotine in any form. The nicotine may act as a stimulant to e.g. obtain a rapid reduction of the urge to smoke or to use tobacco. More particularly, the nicotine should be in a saliva soluble form to facilitate the release of the agent into the saliva in the oral cavity and, further, the subsequent uptake of the nicotine from the saliva in the oral cavity into the systemic circulation of the subject.

With nicotine it is intended to include nicotine, 3-(1-rnethyl-2-pyrrolidinyl)-pyridine, with its base form, including synthetic nicotine as well as nicotine extracts from tobacco plants, or parts thereof, such as the genus Nicotiana alone or in combination or pharmaceutically acceptable salts.

Preferred embodiments incorporate nicotine as (a) the free base form; (b) a water-soluble pharmaceutically acceptable salt, either per se or adsorbed on an adsorbent; (c) a complex with a cation exchanger; (d) mixtures of any of (a)-(c); (e) an inclusion complex, such as a cyclodextrin complex, e. g. 3-cyclodextrin, or nicotine in any non- covalent binding; (0 nicotine bound to zeolites; (g) nicotine bound to cellulose or starch microspheres; and (h) mixtures of any of the foregoing.

Also, any other suitable pharmaceutically acceptable form may also be employed.

As noted above, numerous nicotine salts are known, and may be used. Particular examples of suitable salts include tartrate, hydrogen tartrate, citrate, malate, and/or hydrochloride.

Also useful herein are tooth desensitizing agents. Tooth desensitizing agents that may be used in the present invention include potassium nitrate, citric acid, citric acid salts, strontium chloride, and the like, as well as other desensitizing agents known in the art. One particular embodiment includes a desensitizing agent in combination with a tooth whitening agent. The amount of desensitizing agent included within the dental whitening compositions of the present invention may vary according to the concentration of the potassium nitrates, the desired strength and intended treatment times. Accordingly, if included at all, the other desensitizing agents will preferably be included in an amount in a range from about 0.1% to about 10% by weight of the dental desensitizing composition, more preferably in a range from about 1 to about 7% by weight of the wet sub-coatcomposition.

An individual enzyme or a combination of several compatible enzymes can also be included in the chewing gum composition of the present invention.

Antioxidants are generally recognized as useful in compositions such as those of the present invention. Antioxidants that may be included in the coating compositions of the present invention include, but are not limited to, Vitamin E, ascorbic acid, uric acid, carote- noids, Vitamin A, flavonoids and polyphenols, herbal antioxidants, melatonin, aminoindoles, lipoic acids and mixtures thereof.

It may be desirable to add pH adjusting agents, or buffers, such as sodium bicarbonate, sodium phosphate, sodium hydroxide, ammonium hydroxide, sodium stannate, citric acid, hydrochloric acid, sodium citrate, and combinations thereof to the core and/or to any of the coatings. The pH adjusting agents are added in sufficient amounts so as to adjust the pH of oral cavity to a suitable value, e.g. from about 4.5 to about 11, preferably from about 5.5 to about 8.5.

The particulate material according to the present invention pertains to a BET specific surface area of the inorganic mineral filler is between 15 m²/g and 200 m²/g, the BET specific surface area measured in accordance with ISO 9277.

The particulate material according to the present invention pertains to an average grain diameter of the inorganic mineral filler is between 50 and 0.1 microns.
In a particular embodiment of the invention, the inorganic mineral filler has the following characteristics: a mean grain diameter, measured by the sedimentation method on a Sedigraph 5100™ instrument, between 50 and 0.1 microns and a BET specific surface area, measured in accordance with ISO 9277, ranging from 15 m2/g to 200 m2/g.

In an even more particular manner they are characterised by the fact that inorganic mineral filler has the following characteristics: a mean grain diameter, measured by the sedimentation method on a Sedigraph 5100™ instrument, between 25 and 0.5 microns and even more particularly between 7 and 0.7 microns and a BET specific surface area, measured in accordance with ISO 9277, ranging from 20 m2/g to 80 m2/g and even more particularly between 30 and 60 m2/g.

The particulate material according to one embodiment of the invention, wherein the weight of the dried inorganic mineral filler is increased by at least 1 % at a relative humidity of 95 % at 25 degree Celcius compared to a relative humidity of 0 %, such as 2%, 3%, 4%, 5%, 6%, 7%, 8%, 9%, 10% or 12%.

The particulate material according to the present invention, wherein the powder flow of the particulate material is higher than the powder flow of particulate material with a BET specific surface area of inorganic mineral filler below 15 m²/g, the BET specific surface area measured in accordance with ISO 9277.

In a preferred embodiment of the present invention, the inorganic mineral filler comprises a natural calcium carbonate, such as a marble, a calcite, a chalk or a carbonate containing dolomite; and/or a precipitated calcium carbonate (PCC).

In a preferred embodiment of the present invention the inorganic mineral filler is obtainable by pre-treatment with one or more medium-strong to strong sources of H₃O⁺ ions and/or pre-treatment with an inorganic salt, such as a magnesium sulphate in combination with aluminium sulphate and/or zinc sulphate, and pre-treatment with gaseous CO₂, and for precipitated calcium carbonate preferably at a CO₂ gas flow rate of below 30 litres per minute at standard temperature and pressure per kilogram calcium hydroxide during precipitation.

Accordingly, the natural carbonate acoording to the present invention, such as for example natural calcium carbonate or dolomite, is treated in combination by one or more medium-strong to strong providers of H₃O⁺ ions and gaseous CO2.

Various natural carbonates may be suitable obtainable from chalk, in particular chalk from Champagne, calcite or marble, and mixtures thereof with talc, kaolin and/or dolomite, and/or hydroxides of aluminium, and/or titanium oxide, magnesium oxide and similar oxides and hydroxides known in the industry concerned.

The invention particularly concerns the treatment, by a combination of one or more medium-strong to strong H₃O⁺ ion-providers in an active gaseous medium, of inorganic mineral filler, containing natural carbonate such as natural calcium carbonate, and/or in combination with other minerals.

The acid used will be any medium-strong or strong acid or any mixture of such acids, generating H3O+ ions under the processing conditions.

In one embodiment it is preferred, that the strong acid will be chosen among the acids with a pKa value lower than or equal to zero at 22° C. and more particularly chosen from sulphuric acid, hydrochloric acid or mixtures thereof.

In another embodiment it is preferred, that the medium-strong acid will be chosen among the acids with a pKa value between 0 and 2.5 inclusive at 22° C. and more particularly chosen from H2SO4, HSO4-, H3PO4 and oxalic acid or mixtures thereof. We can quote as a particular example a pKa1 of H3PO4 equal to 2.161 (Rompp Chemie, Edition Thieme).

In one embodiment of the invention it is preferred, that the medium-strong acid or acids can be mixed with the strong acid or acids.

According to the invention, the molar quantity of medium-strong to strong providers of H3O+ ions relative to the number of moles of CaCO3 is in total between 0.1 and 2 and preferably between 0.25 and 1.

According to the invention, the process is characterised by the fact that the said filler is treated by a combination of one or more medium-strong to strong providers of H3O+ ions and gaseous CO2.

The particulate material according to the present invention may further comprise a modifying agent, such as a water-soluble natural or synthetic polymer.

Polymers suitable as modifying agent are preferably selected from, but not limited to, the group consisting of hydroxypropyl cellulose, hydroxypropylmethyl cellulose, hydroxyethyl cellulose, polyvinyl pyrrolidone, carboxymethyl cellulose, polyvinyl alcohol, sodium alginate, polyethylene glycol, pullulan, tragacanth gum, guar gum, acacia gum, arabic gum, polyacrylic acid, methylmethacrylate copolymer, carboxyvinyl polymer, amylose, high amylose starch, hydroxypropylated high amylose starch, dextrin, pectin, chitin, chitosan, gelatin, zein, gluten, soy protein isolate, whey protein- isolate, casein and mixtures thereof. Suitable polymers also include water- insoluble polymers selected from the group consisting of hydrogenated vegetable oils, hydrogenated caster oil, polyvinyl chloride, shellac, polyurethane, cellulose derivatives, gum rosins, wood rosens, waxes, acrylate and methacrylate polymers, copolymers of acrylic and methacrylic acid esters and mixtures thereof.

The particulate material according to the present invention may be used in a chewing gum, compressed chewing gum, a mouth spray, a nasal spray, an inhaling device, a tablet, such as a sublingual tablet, a lozenge, a buccal sachet, a transdermal patch or a powder.

In particular, the present invention relates to a chewing gum comprising the particulate material according to the present invention.

The chewing gum may comprise a chewing gum core. The chewing gum core may comprise a continuous mass of pre-heated chewing gum ingredients, including gum base. The chewing gum may also comprise a compressed mixture of chewing gum granules, including gum base.

In one embodiment of the invention the particulate material is contained in the chewing gum core. In another embodiment of the invention the particulate material is part of a sub-coat between the chewing gum core and an outer coating. In yet another embodiment the chewing gum comprise a combination of the above.

Preferred embodiments contain nicotine in an amount of 0.5-6 mg per tablet calculated as free base/unit dose.

In one embodiment, the particulate material is used in chewing gum. A chewing gum product according to the present invention may be a medicated chewing gum. Medicated chewing gums are herein intended to mean solid or semi-solid, single-dose preparations with a base consisting mainly of gum that are intended to be chewed but not swallowed, whereby the chewing gum acts as a drug delivery system. Such gums contain one or more active substances, which are released upon chewing. After dissolution or dispersion of the active substance in the saliva systemic delivery of the drug takes place through kansmucosal uptake throughout the oral cavity.

Preferred embodiments may contain nicotine in an amount of 0.5 - 6 mg calculated as the amount of free base of nicotine per piece chewing gum product.

Optional addition of buffering agents in any of the delievery systems above may be buffering agents, optionally added mainly, but not exclusively, in formulations of the present invention intended for buccal delivery.

Absorption of nicotine from the oral cavity to the systemic circulation is dependent on the pH of the saliva and the plea of nicotine, which is about 7.8. Assuming a pH of the saliva of 6.8 only about 10% of the nicotine in saliva will be in the free base form. Thus, in order to promote absorption of nicotine in a free base form, which is the form predomi- nantly absorbed through the mucosa, the pH of the saliva must be increased. At a pH of 8.8 about 90% of the nicotine in saliva will then be in the free base form.

Hence and according to the invention, the present nicotine- containing pharmaceutical composition may be alkalised by buffering andlor pH regulation. This may be achieved by including physiologically acceptable buffering substances or agents, or by other means. With other means it is intended to include buffering by any component in the product, which may not normally act as a buffering agent, such as a self- buffering additive and/or pH regulating forms of nicotine.

By buffering andlor pH regulation thereby increasing the pH of the saliva the uptake of nicotine is changed, e g increased compared to the nicotine uptake when the saliva is not aLkalised by buffering andlor pH regulation. Also, since the transmucosal uptake of nicotine in the oral cavity according to the invention is faster than for nicotine not being buffered andlor pH regulated according to the invention, less nicotine will be swallowed and reach the gastrointestinal (GI) tract. The nicotine that reaches the GI tract will be subjected to first pass metabolism, which reduces the total amount of intact nicotine absorbed. This means that the bioavailability of nicotine that is not co-administered with a buffer according to the invention will generally be lower than when administered together with a buffer.

For buffering may be used one or more buffering agents selected from the group consisting of carbonates including bicarbonate or sesquicarbonate7 glycinate, phosphate, glycerophosphate or citrate of an alkali metal, such as potassium or sodium, or ammonium; sodium hydroxide, potassium hydroxide, calcium oxide, and mixtures thereof.

Further embodiments may use trisodium or tripotassium citrate, and mixtures thereof.

Still further embodiments may comprise different phosphate systems, such as trisodium phosphate, disodium hydrogen phosphate; and tripotassium phosphate, dipo- tassium hydrogen phosphate, and calcium hydroxide, sodium glycinate; and mixtures thereof.

Alkali metal carbonates, glycinates and phosphates are preferred buffering agents. The pH regulation may also be obtained by using pH-regulating forms of nicotine, e g nicotine free base.

The amount of the buffering agent or agents in the liquid pharmaceutical formulation is preferably sufficient in the specific embodiments to raise the pH of the saliva to above 7, as specified above and, to maintain the pH of the saliva in the oral cavity above 7, e g pH 7 - 11. Otherwise expressed the liquid pharmaceutical formulation should be alkalised by buffering and/or pH regulation in such a way that upon administration to a subject the pH of the liquid of the oral cavity of the subject is transiently increased by about 0.3 - 4 pH units, preferably by about 0.5 - 2.5 pH units. The amount of buffering agent(s) required to achieve such an increase in pH is readily calculated by a person skilled in the art.

Other additives may be added optionally to the present nicotine-containing compo sition.

Optional additives comprise one or more stabilizing additives, such as those selec ted from the group consisting of antioxidants including vitamin E, i e tocopheroles, vitamin C, i e ascorbic acid and its salts, sodium pyrosulfite, butylhydroxytoluene, butylated hyd roxyanisole; and preservatives including parabenes, benzalkonium chloride, chlorbutanol, benzyl alcohol, beta-phenylethyl alcohol, cetylpyridinium chloride, citric acid, tartaric acid, lactic acid, malic acid, acetic acid, benzoic acid, and sorbic acid and their salts; and chelating agents, such as EDTA; and galates, such as propyl galate.

Further optional additives comprise one or more additives selected from the group consisting of: - enhancers, such as ozone; - vitamins, such as vitamins B. C and E; - minerals, such as fluorides, especially sodium fluoride, sodium monofluoro phosphate and stannous fluoride; - anti- odours, such as zinc and cyclodextrins; - propellants, such as 1,1, 2,2- tekafluoroethane (HFC-134a), optionally being liquefied, and 1,1,1,2, 3,3, 3-heptafluororpropane (HFC-227), optionally being liquefied; - sweeteners including one or more synthetic sweetening agents and/or natural sugars, such as those selected from the groups consisting of e g saccharin and its sodium and calcium salts, aspartame, acesulfame and its potassium salt, thaumatin, glycyrrhizin, sucralose, dihydrochalcone, alitame, miraculin, monellin and stevside.
- polyhydric alcohols such as sorbitol, xylitol, mannitol and glycerol; - monosaccharides including glucose (also called dextrose), fructose (also called laevulose) and galactose; - disaccharides including saccharose (also called sucrose), lactose (also called milk sugar) and maltose (also called malt sugar); - mixtures of sugars including liquid glucose syrup e g starch hydrolysates con- taining a mixture of chiefly dextrose, maltose, dextrins and water, invert sugar syrup e g sucrose inverted by invertase containing a mixture of dextrose, laevulose and water, high sugar content syrups such as treacle, honey and malt extract; and mixtures thereof; - flavoring and/or aromatizing agents, such as those selected from the group consisting of essential oils obtained by distillations, solvent extractions or cold expressions of fresh or dried flowers, buds, leaves, stems, fruit, seeds, peel, bark, or root e g oil of pepper- mint, spearmint, eucalyptus, wintergreen, niaouli, clove, cardamom, cinnamon, bitter almond, coriander, caraway, ginger, juniper, orange, bitter orange, lemon, grapefruit, mandarine, bergamot, thyme, fennel and rosemary; - natural flavors and aroma agents including either diluted solutions of essential oils or concentrates of flavor components with natural origin from e g fruits, berries, nuts, spices, mints, tobacco, cocoa, coffee, tea, vanilla, liquorice, caramel, toffee, honey, wine, liquors and brews; - synthetic flavors and aroma agents consisting of mixtures of chemicals com- prising hydrocarbons, alcohols, aldehydes, esters, ketones, ethers and oxides blended to match the natural flavor of e g fruits, berries, nuts, spices, mints, tobacco, cocoa, coffee, tea, vanilla, liquorice, caramel, toffee, honey, wine, liquors or brews; - and mixtures thereof.

Thus, in one aspect the invention relates to a nicotine-containing particulate material for release of nicotine in chewing gum, the material comprising a combination of nicotine or a pharmaceutically acceptable salt, complex or solvate thereof and a microcrystalline cellulose, the particulate material - when tested in an in vitro dissolution test - releasing at least 90% w/w such as, e.g. at least 95 % w/w of the nicotine or the pharmaceutically acceptable salt, complex or solvate thereof within at the most about 30 min such as, e.g., at the most about 25 min, at the most about 20 min, at the most about 15 min, at the most about 10 min, at the most about 7.5 min, at the most about 5 min, at the most about 4 min, at the most about 3 min or at the most about 2 min.

More specifically, the invention may relate to a particulate material for fast release of nicotine in chewing gum, the material - when tested in an in vitro dissolution test - releasing at least 90% w/w such as, e.g. at least 95 % w/w of the nicotine or the pharmaceutically acceptable salt, complex or solvate thereof within at the most about 20 min such as, e.g, at the most about 15 min, at the most about 10 min, at the most about 7.5 min, at the most about 5 min, at the most about 4 min, at the most about 3 min, at the most about 2 min or at the most about 1 min.

In one embodiment of the invention the chewing gum core comprises a continuous mass of non-particular gum base, and/or a compressed mixture of chewing gum granules including gum base.

In one embodiment of the invention the particulate material is contained in a sub-layer between the chewing gum core and an outer coating.

According to the present invention, flavoring agents may be incorporated in the core or in the sub-layer between the core and an outer coating.. Advantages of the invention include long lasting effect of flavoring agent(s), domination of flavoring agents in the coating(s) over flavoring agent(s) in the core, the avoidance of problems of chemical or pharmaceutical incompatibility between a drug in the core and flavoring agent(s) in the coating(s), and increased control of the release of the drug and of non-active excipients.

In one embodiment the particulate material is contained in the chewing gum core.

In one embodiment the particulate material is contained in an outer coating.

Further, the present invention relates to a method of manufacture a chewing gum comprising the steps of:
i) preparing a combination of an active ingredient being nicotine and an inorganic mineral filler, wherein the active ingredient is reversibly absorbed into and/or adsorbed onto the inorganic mineral filler, obtaining a particulate material, wherein the particulate material comprises a combination of one or more active ingredients, such as nicotine or a flavoring agent, and an inorganic mineral filler, and wherein the BET specific surface area of the inorganic mineral filler is above 15 m²/g, the BET specific surface area measured in accordance with ISO 9277,
ii) formulating the particulate material with chewing gum ingredients, including gum base, obtaining a chewing gum having controlled release of the active ingredient, and
iii) optionally, pre-treating the inorganic mineral filler prior to step i) with one or more medium-strog to strong providers of H₃O⁺ ions and/or pre-treated with an inorganic salt, such as a magnesium sulphate in combination with aluminium sulphate and/or zinc sulphate, and pre-treated with gaseous CO₂, and for precipitated calcium carbonate preferably at a CO₂ gas flow rate of below 30 litres per minute at standard temperature and pressure per kilogram calcium hydroxide during precipitation.

In a preferred manner this process according to the invention is characterised by:
a) Treatment with one or more medium-strong to strong providers of H3O+ ions b) Treatment with gaseous CO2, whether this treatment be an integral part of stage a), be carried out in parallel with stage a) or be carried out after stage a) c) The raising of pH beyond 7.5, measured at 20° C., in a time interval after the end of stages a) and b) of between 1 hour and 10 hours and preferably between 1 hour and 5 hours without addition of a base, or immediately after the end of stages a) and b) with the addition of a base, stage c) being the final stage in the process.

In a preferred manner also, the gaseous CO2 comes from an external CO2 supply or from the recirculation of CO2 or from the continuous addition of the same medium-strong to strong provider of H3O+ ions as used in stage a) of the treatment or from another medium-strong to strong provider of H3O+ ions or from an excess pressure of CO2, preferably an excess pressure of between 0.05 and 5 bars. In this regard, it should be noted that the processing tank, filled with fillers having a specific gravity of the order of 1 to 2, may reach a height of for example 20 metres and hence create an excess pressure of CO2 which can reach several bars and in particular up to approximately 5 bars at the bottom of the tank or in a closed tank.

In a preferred mode of implementation, stages a) and b) may be repeated several times.

Similarly, in a preferred mode of implementation, the pH measured at 20° C. ranges from 3 to 7.5 during stages a) and b) of processing and the processing temperature is between 50° C. and 90° C. and preferably between 45° C. and 60° C.

In another preferred mode of implementation, between 1 hour and 10 hours and more particularly between 1 hour and 5 hours after the end of processing, the pH is greater than 7.5 at ambient temperature without the addition of any base whatever. If any base is added, the pH then rises immediately. It should moreover be noted that after several days no resistance to acids is observed.

The process in one embodiment is characterised by the fact that the concentration of gaseous CO2 in the suspension is, in terms of volume, such that the ratio (volume of suspension:volume of gaseous CO2) is between 1:0.05 and 1:20 with the said ratio being between 1:1 and 1:20 in stage a) and between 1:0.05 and 1:1 in stage b).

In a highly preferable manner, the concentration of gaseous CO2 in the suspension is, in terms of volume, such that the ratio (volume of suspension:volume of gaseous CO2) is between 1:0.05 and 1:5 with the said ratio being between 1:0.5 and 1:10 in stage a) and between 1:0.05 and 1:1 in stage b).

The gaseous CO2 may be introduced in liquid or anhydride form.

In a manner which is also preferred, the duration of stage b) of the treatment is from 0 to 10 hours and preferably from 2 to 6 hours.

The treatment process according to the invention is implemented in the aqueous (slurry) phase at low, medium-high or high concentrations of dry matter, but can also be implemented for mixtures of slurries at those differing concentrations. In a preferential manner, the dry matter content by weight is between 1% and 80%.

Without wishing to be bound by any theory, the applicant believes that the gaseous CO2 plays the part, among others, of a pH regulator and a regulator of adsorption/desorption.

In one embodiment the amount of gum base according to the invention is about 15 - 80 % by weight of the total gum core, and preferably at least about 40 % by weight.

The gum base may be of any conventional nature known in the art. For example it may comprise a gum base of natural or synthetic origin readily available from a commercial source. Natural gum bases include e. g. chicle, jelutong-, lechi de caspi-, soh-, siak-, katiau-, sorwa-, balata-, pendare-, malaya-, and peach gums, natural cautchouc and natural resins such as dammar and mastix. Synthetic gum bases are a mixture of: - elastomers (polymers, masticating substances), - plasticizer (resin, elastomers, solvent, hydrophobic resin), - filler (texturizer, water- insoluble adjuvant), - softener (fat), - emulsifier, - wax, - antioxidant, and - anti-tacking agents (vinyl polymer, hydrophilic resin).

Other examples of gum bases are gums including agar, alginate, arabic gum, carob gum, carrageenan, ghatti gum, guar gum, karaya gum, pectin, tragacanth gum, locust beam gum, gellan gum and xanthan gum.

Examples of gelling agents comprise gum arabic, starch, gelatine, agar, and pectin.

When nicotine in any form is incorporated in the chewing gum mass in accordance with the present invention, it is possible to employ a wide variety of chewing gum compositions and amounts of the chewing gum base. Different chewing gum products may be composed depending on the consumer's preference and the purpose of use, in respect of the nicotine level, nicotine distribution and other additives.

In an embodiment of the invention, said natural resin comprises terpene resins, e.g. derived from alpha-pinene, beta-pinene, and/or d-limonene, natural terpene resins, glycerol esters of gum rosins, tall oil rosins, wood rosins or other derivatives thereof such as glycerol esters of partially hydrogenated rosins, glycerol esters of polymerized rosins, glycerol esters of partially dimerised rosins, pentaerythritol esters of partially hydrogenated rosins, methyl esters of rosins, partially hydrogenated methyl esters of rosins or pentaerythritol esters of rosins and combinations thereof.

Materials to be used for the above-mentioned encapsulation methods might e.g. include Gelatine, Wheat protein, Soya protein, Sodium caseinate, Caseine, Gum arabic, Mod. starch, Hydrolyzed starches (maltodextrines), Alginates, Pectin, Carregeenan, Xanthan gum, Locus bean gum, Chitosan, Bees wax, Candelilla wax, Carnauba wax, Hydrogenated vegetable oils, Zein and/or Sucrose.

Examples of generally synthetic resins include polyvinyl acetate, vinyl acetate-vinyl laurate copolymers and mixtures thereof. Examples of non-biodegradable synthetic elastomers include, but are not limited to, synthetic elastomers listed in Food and Drug Administration, CFR, Title 21, Section 172,615, the Masticatory Substances, Synthetic) such as polyisobutylene. e.g. having a gel permeation chromatography (GPC) average molecular weight in the range of about 10,000 to 1,000,000 including the range of 50,000 to 80,000, isobutylene-isoprene copolymer (butyl elastomer), styrene-butadiene copolymers e.g. having styrene-butadiene ratios of about 1:3 to 3:1, polyvinyl acetate (PVA), e.g. having a GPC average molecular weight in the range of 2,000 to 90,000 such as the range of 3,000 to 80,000 including the range of 30,000 to 50,000, where the higher molecular weight polyvinyl acetates are typically used in bubble gum base, polyisoprene, polyethylene, vinyl acetate-vinyl laurate copolymer e.g. having a vinyl laurate content of about 5 to 50% by weight such as 10 to 45% by weight of the copolymer, and combinations hereof.
The elastomers (rubbers) employed in the gum base may vary depending upon various factors such as the type of gum base desired, the texture of gum composition desired and the other components used in the composition to make the final chewing gum product. The elastomer may be any water-insoluble polymer known in the art, and includes those gum polymers utilized for chewing gums and bubble gums. Illustrative examples of suitable polymers in gum bases include both natural and synthetic elastomers. For example, those polymers which are suitable in gum base compositions include, without limitation, natural substances (of vegetable origin) such as chicle gum, natural rubber, crown gum, nispero, rosidinha, jelutong, perillo, niger gutta, tunu, balata, guttapercha, lechi capsi, sorva, gutta kay, and the like, and mixtures thereof. Examples of synthetic elastomers include, without limitation, styrene-butadiene copolymers (SBR), polyisobutylene, isobutylene-isoprene copolymers, polyethylene, polyvinyl acetate and the like, and mixtures thereof.

It is common in the industry to combine in a gum base a synthetic elastomer having a high molecular weight and a synthetic elastomer having a low molecular weight. Examples of such combinations are polyisobutylene and styrene-butadiene, polyisobutylene and polyisoprene, polyisobutylene and isobutylene-isoprene copolymer (butyl rubber) and a combination of polyisobutylene, styrene-butadiene copolymer and isobutylene isoprene copolymer, and all of the above individual synthetic polymers in admixture with polyvinyl acetate, vinyl acetate-vinyl laurate copolymers, respectively and mixtures thereof.

Examples of natural resins are: Natural rosin esters, often referred to as ester gums including as examples glycerol esters of partially hydrogenated rosins, glycerol esters of polymerised rosins, glycerol esters of partially dimerized rosins, glycerol esters of tally oil rosins, pentaerythritol esters of partially hydrogenated rosins, methyl esters of rosins, partially hydrogenated methyl esters of rosins, pentaerythritol esters of rosins, synthetic resins such as terpene resins derived from alpha-pinene, beta-pinene, and/or d-limonene, and natural terpene resins.

The chewing gum according to the invention may be provided with an outer coating.

The applicable hard coating may be selected from the group comprising of sugar coating and a sugarless coating and a combination thereof. The hard coating may e.g. comprise 50 to 100% by weight of a polyol selected from the group consisting of sorbitol, maltitol, mannitol, xylitol, erythritol, lactitol and Isomalt and variations thereof. In an embodiment of the invention, the outer coating is an edible film comprising at least one component selected from the group consisting of an edible film-forming agent and a wax. The film-forming agent may e.g. be selected from the group comprising cellulose derivative, a modified starch, a dextrin, gelatine, shellac, gum arabic, zein, a vegetable gum, a synthetic polymer and any combination thereof.

In an embodiment of the invention, the outer coating comprises at least one additive component selected from the group comprising of a binding agent, a moisture-absorbing component, a film-forming agent, a dispersing agent, an antisticking component, a bulking agent, a flavoring agent, a coloring agent, a pharmaceutically or cosmetically active component, a lipid component, a wax component, a sugar, an acid and an agent capable of accelerating the after-chewing degradation of the degradable polymer.

Generally, the ingredients may be mixed by first melting the gum base and adding it to the running mixer. Colors, active agents and/or emulsifiers may also be added at this time. A softener such as glycerin may also be added at this time, along with syrup and a portion of the bulking agent/sweetener. Further portions of the bulking agent/sweetener may then be added to the mixer. A flavoring agent is typically added with the final portion of the bulking agent/sweetener. A high-intensity sweetener is preferably added after the final portion of bulking agent and flavor has been added.

The entire mixing procedure typically takes from five to fifteen minutes, but longer mixing times may sometimes be required. Those skilled in the art will recognize that many variations of the above-described procedure may be followed. Including the one-step method described in US patent application 2004/0115305 hereby incorporated as reference. Chewing gums are formed by extrusion, compression, rolling and may be centre filled with liquids and/or solids in any form.

The chewing gum according to the present invention may also be provided with an outer coating, which may be a hard coating, a soft coating, a film coating, or a coating of any type that is known in the art, or a combination of such coatings. The coating may typically constitute 0.1 to 75% by weight of a coated chewing gum piece.

One preferred outer coating type is a hard coating, which term is including sugar coatings and sugar-free (or sugarless) coatings and combinations thereof. The object of hard coating is to obtain a sweet, crunchy layer, which is appreciated by the consumer and to protect the gum centers. In a typical process of providing the chewing gum centers with a protective sugar coating the gum centers are successively treated in suitable coating equipment with aqueous solutions of crystallizable sugar such as sucrose or dextrose, which, depending on the stage of coating reached, may contain other functional ingredients, e.g. fillers, colors, etc.

In one presently preferred embodiment, the coating agent applied in a hard coating process is a sugarless coating agent, e.g. a polyol including as examples sorbitol, maltitol, mannitol, xylitol, erythritol, lactitol and isomalt or e.g. a mono- di-saccharide including as example trehalose.

Or alternatively a sugar-free soft coating e.g. comprising alternately applying to the centers a syrup of a polyol or a mono- di-saccharide, including as examples sorbitol, maltitol, mannitol, xylitol, erythritol, lactitol, isomalt and trehalose.

In further useful embodiments, a film coating is provided by film-forming agents such as a cellulose derivative, a modified starch, a dextrin, gelatine, zein, shellec, gum arabic, a vegetable gum, a synthetic polymer, etc. or a combination thereof.

In an embodiment of the invention, the outer coating comprises at least one additive component selected from the group comprising a binding agent, a moisture-absorbing component, a film-forming agent, a dispersing agent, an antisticking component, a bulking agent, a flavoring agent, a coloring agent, a pharmaceutically or cosmetically active component, a lipid component, a wax component, a sugar, and an acid.

A coated chewing gum center according to the invention may have any form, shape or dimension that permits the chewing gum center to be coated using any conventional coating process.

It should however be noted that application of different coating should be done with care as compressed chewing gum tablets may be very affected by direct contact with moisture or water.

The composition of gum base formulations can vary substantially depending on the particular product to be prepared and on the desired masticatory and other sensory characteristics of the final product. However, typical ranges of the above gum base components are: 5 to 80% by weight of elastomeric compounds, 5 to 80% by weight of elastomer plasticizers, 0 to 40% by weight of waxes, 5 to 35% by weight of softener, 0 to 50% by weight of filler, and 0 to 5% by weight of miscellaneous ingredients such as antioxidants, colorants, etc. The gum base may comprise about 5 to about 95% by weight of the chewing gum, more commonly; the gum base comprises 10 to about 60% by weight of the gum.

Elastomers provide the rubbery, cohesive nature to the gum, which varies depending on this ingredient's chemical structure and how it may be compounded with other ingredients. Elastomers suitable for use in the gum base and gum of the present invention may include natural or synthetic types.

Elastomer plasticizers vary the firmness of the gum base. Their specificity on elastomer inter-molecular chain breaking (plasticizing) along with their varying softening points cause varying degrees of finished gum firmness and compatibility when used in gum base. This may be important when one wants to provide more elastomeric chain exposure to the alkanic chains of the waxes.

If desired, conventional elastomers or resins may be supplemented or substituted by biodegradable polymers.

In addition to a water insoluble gum base portion, a typical chewing gum includes a water soluble bulk portion and one or more flavoring agents. The water-soluble portion may include bulk sweeteners, high-intensity sweeteners, flavoring agents, softeners, emulsifiers, colors, acidulants, buffering agents, fillers, antioxidants, and other components that provide desired attributes.

Combinations of sugar and/or non-sugar sweeteners can be used in the chewing gum formulation processed in accordance with the invention. Additionally, the softener may also provide additional sweetness such as aqueous sugar or alditol solutions.

Useful sugar sweeteners are saccharide-containing components commonly known in the chewing gum art including, but not limited to, sucrose, dextrose, maltose, dextrins, trehalose, D-tagatose, dried invert sugar, fructose, levulose, galactose, corn syrup solids, and the like, alone or in combination.

Sorbitol can be used as a non-sugar sweetener. Other useful non-sugar sweeteners include, but are not limited to, other sugar alcohols such as mannitol, xylitol, hydrogenated starch hydrolysates, maltitol, isomaltol, erythritol, lactitol and the like, alone or in combination.

High-intensity artificial sweetening agents can also be used alone or in combination with the above sweeteners. Preferred high-intensity sweeteners include, but are not limited to sucralose, aspartame, salts of acesulfame, alitame, neotame, twinsweet, saccharin and its salts, cyclamic acid and its salts, glycyrrhizin, dihydrochalcones, thaumatin, monellin, stevioside and the like, alone or in combination. In order to provide longer lasting sweetness and flavor perception, it may be desirable to encapsulate or otherwise control the release of at least a portion of the artificial sweetener. Techniques such as wet granulation, wax granulation, spray drying, spray chilling, fluid bed coating, coascervation, encapsulation in yeast cells and fiber extrusion may be used to achieve the desired release characteristics. Encapsulation of sweetening agents can also be provided using another chewing gum component such as a resinous compound.

Usage level of the high-intensity artificial sweetener will vary considerably and will depend on factors such as potency of the sweetener, rate of release, desired sweetness of the product, level and type of flavor used and cost considerations. Thus, the active level of high-potency artificial sweetener may vary from about 0 to about 8% by weight, preferably 0.001 to about 5% by weight. When carriers used for encapsulation are included, the usage level of the encapsulated sweetener will be proportionately higher.

If a low-calorie gum is desired, a low-caloric bulking agent can be used. Examples of low caloric bulking agents include polydextrose, Raftilose, Raftilin, fructooligosaccharides (NutraFlora®), palatinose oligosaccharides; guar gum hydrolysates (e.g. Sun Fiber®) or indigestible dextrins (e.g. Fibersol®). However, other low-calorie bulking agents can be used.

The chewing gum according to the present invention may contain aroma agents and flavoring agents including natural and synthetic flavorings e.g. in the form of natural vegetable components, essential oils, essences, extracts, powders, including acids and other substances capable of affecting the taste profile. Examples of liquid and powdered flavorings include coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, and mint, fruit essence such as from apple, pear, peach, strawberry, apricot, raspberry, cherry, pineapple, and plum essence. The essential oils include peppermint, spearmint, menthol, eucalyptus, clove oil, bay oil, anise, thyme, cedar leaf oil, nutmeg, and oils of the fruits mentioned above.

The chewing gum flavor may be a natural flavoring agent, which is freeze-dried, preferably in the form of a powder, slices or pieces or combinations thereof. The particle size may be less than 3 mm, less than 2 mm or more preferred less than 1 mm, calculated as the longest dimension of the particle. The natural flavoring agent may be in a form where the particle size is from about 3 µm to 2 mm, such as from 4 µm to 1 mm. Preferred natural flavoring agents include seeds from fruit e.g. from strawberry, blackberry and raspberry.

Various synthetic flavors, such as mixed fruit flavors may also be used in the present chewing gum centers. As indicated above, the aroma agent may be used in quantities smaller than those conventionally used. The aroma agents and/or flavors may be used in the amount from 0.01 to about 30% by weight of the final product depending on the desired intensity of the aroma and/or flavor used. Preferably, the content of aroma/flavor is in the range of 0.2 to 3% by weight of the total composition.

In an embodiment of the invention, the flavoring agents comprise natural and synthetic flavorings in the form of natural vegetable components, essential oils, essences, extracts, powders, including acids and other substances capable of affecting the taste profile.

In one embodiment of the invention, the flavor may be used as taste masking in chewing gum comprising active ingredients, which by themselves have undesired taste or which alter the taste of the formulation.

Further chewing gum ingredients, which may be included in the chewing gum according to the present invention, include surfactants and/or solubilisers, especially when pharmaceutically or biologically active ingredients are present. As examples of types of surfactants to be used as solubilisers in a chewing gum composition according to the invention, reference is made to H.P. Fiedler, Lexikon der Hilfstoffe für Pharmacie, Kosmetik und Angrenzende Gebiete, pages 63-64 (1981) and the lists of approved food emulsifiers of the individual countries. Anionic, cationic, amphoteric or non-ionic solubilisers can be used. Suitable solubilisers include lecithin, polyoxyethylene stearate, polyoxyethylene sorbitan fatty acid esters, fatty acid salts, mono and diacetyl tartaric acid esters of mono and diglycerides of edible fatty acids, citric acid esters of mono and diglycerides of edible fatty acids, saccharose esters of fatty acids, polyglycerol esters of fatty acids, polyglycerol esters of interesterified castor oil acid (E476), sodium stearoyllatylate, sodium lauryl sulfate and sorbitan esters of fatty acids and polyoxyethylated hydrogenated castor oil (e.g. the product sold under the trade name CREMOPHOR), block copolymers of ethylene oxide and propylene oxide (e.g. products sold under trade names PLURONIC and POLOXAMER), polyoxyethylene fatty alcohol ethers, polyoxyethylene sorbitan fatty acid esters, sorbitan esters of fatty acids and polyoxyethylene steraric acid esters.

Particularly suitable solubilisers are polyoxyethylene stearates, such as for instance polyoxyethylene(8)stearate and polyoxyethylene(40)stearate, the polyoxyethylene sorbitan fatty acid esters sold under the trade name TWEEN, for instance TWEEN 20 (monolaurate), TWEEN 80 (monooleate), TWEEN 40 (monopalmitate), TWEEN 60 (monostearate) or TWEEN 65 (tristearate), mono and diacetyl tartaric acid esters of mono and diglycerides of edible fatty acids, citric acid esters of mono and diglycerides of edible fatty acids, sodium stearoyllatylate, sodium laurylsulfate, polyoxyethylated hydrogenated castor oil, blockcopolymers of ethylene oxide and propyleneoxide and polyoxyethylene fatty alcohol ether. The solubiliser may either be a single compound or a combination of several compounds. In the presence of an active ingredient, the chewing gum may preferably also comprise a carrier known in the art.

Further active ingredients may advantageously be applied in a chewing gum according to the invention. Further active ingredients generally refer to those ingredients that are included in a delivery system and/or compressible chewing gum composition for the desired end benefit they provide to the user. In some embodiments, active ingredients can include medicaments, nutrients, nutraceuticals, herbals, nutritional supplements, pharmaceuticals, drugs, and the like and combinations thereof. Moreover, in the present context, active ingredients may refer to flavor components, high intensity sweeteners or other taste establishing components.

Further active ingredients may be classified according to The Anatomical Therapeutic Chemical (ATC) classification system, which is a system for classification of medicinal products according to their primary constituent and to the organ or system on which they act and their chemical, pharmacological and therapeutic properties.

The first level of the ATC is split into 14 main groups based on the anatomical group:
A: Alimentary tract and metabolism
B: Blood and blood forming organs
C: Cardiovascular system
D: Dermatologicals
G: Genito urinary system and sex hormones
H: Systemic hormonal preparations, excl. sex hormones and insulins
J: Antiinfectives for systemic use
L: Antineoplastic and immunomodulating agents
M: Musculo-skeletal system
N: Nervous system
P: Antiparasitic products, insecticides and repellents
R: Respiratory system
S: Sensory organs
V: Various

Further subdivision is done into a second, third, fourth and fifth sub-group, which is based on the therapeutic main group, the therapeutic/pharmacological subgroup, the chemical/therapeutic/pharmacological subgroup, and the chemical substance subgroup respectively. In this sense each active ingredient has been given a unique ATC identification code indicating where the active ingredient may be useful.

However, as some active ingredients are useful in more than one area, some of the active ingredients mentioned in this document belong to two or more of the mentioned groups, e.g. phenylephrine, which has an ATC identification code in both C, R, and S, i.e. both C01CA06, R01AA04, R01AB01, R01BA03, S01FB01, and S01GA05 are ATC identification codes identifying phenylephrine.

The following list discloses examples of active ingredients which can be classified according to the ATC classification mentioned above and which are active ingredients which may be used in a chewing gum granule or a compressed chewing gum according to the invention:
Ephedrine, Magaldrate, Pseudoephedrine, Sildenafil, Xylocaine, Benzalconium chloride, Caffeine, Phenylephrine, Amfepramone, Orlistat, Sibutramine, Acetaminophen, Aspirin, Aluminum amino acetate, Aluminum amino acetate in combination with Magnesium oxide, Aluminum oxide hydrate in combination with Magnesiumoxide, Calcium carbonate in combination with Magnesium hydroxide, Calciumcarbonate, Dihydroxy Aluminum sodium carbonate, Magnesiumoxide, Glitazones, Metformin, Chlorpromazine, Dimenhydrinat, Domperidone, Meclozine, Metoclopramide, Odansetron, Prednisolone, Promethazine, Acrivastine, Cetirizine, Cinnarizine, Clemastine, Cyclizine, Desloratadine, Dexchlorpheniramine, Dimenhydrinate, Ebastine, Fexofenadine, Ibuprofen, Levolevoproricin, Loratadine, Meclozine, Mizolastine, Promethazine, Miconazole, Vitamin B12, Folic acid, Ferro compounds, vitamin C, Chlorhexidine diacetate, Fluoride, Decapeptide KSL, Aluminum fluoride, Aminochelated calcium, Ammonium fluoride, Ammonium fluorosilicate, Ammonium monofluorphosphate, Calcium fluoride, Calcium gluconate, Calcium glycerophosphate, Calcium lactate, Calcium monofluorphosphate, Calciumcarbonate, Carbamide, Cetyl pyridinium chloride, Chlorhexidine, Chlorhexidine digluconate, Chlorhexidine Chloride, Chlorhexidine diacetate, CPP Caseine Phospho Peptide, Hexetedine, Octadecentyl Ammonium fluoride, Potasium fluorosilicate, Potassium Chloride, Potassium monofluorphosphate, Sodium bi carbonate, Sodium carbonate, Sodium fluoride, Sodium fluorosilicate, Sodium monofluorphosphate, Sodium tri polyphosphate, Stannous fluoride, Stearyl Trihydroxyethyl Propylenediamine Dihydrofluoride, Strontium chloride, Tetra potassium pyrophosphate, Tetra sodium pyrophosphate, Tripotassium orthophosphate, Trisodium orthophosphate, Alginic acid, Aluminum hydroxide, Sodium bicarbonate, Sildenafil, Tadalafil, Vardenafil, Yohimbine, Cimetidine, Nizatidine, Ranitidine, Acetylsalicylic acid, Clopidogrel, Acetylcysteine, Bromhexine, Codeine, Dextromethorphan, Diphenhydramine, Noscapine, Phenylpropanolamine, vitamin D, Simvastatin, Bisacodyl, Lactitol, Lactulose, Magnesium oxide, Sodium picosulfate, Senna glycosides, Benzocaine, Lidocaine, Tetracaine, Almotriptan, Eletriptan, Naratriptan, Rizatriptan, Sumatriptan, Zolmitriptan, Calcium, Chromium, Copper, Iodine, Iron, Magnesium, Manganese, Molybdenium, Phosphor, Selenium, Zinc, Nicotine, Nicotine bitartrate, Nicotine pftalate, Nicotine polacrilex, Nicotine sulphate, Nicotine tartrate, Nicotine citrate, Nicotine lactate, Chloramine, Hydrogenperoxide, Metronidazole, Triamcinolonacetonide, Benzethonium Chl., Cetyl pyrid. Chl., Chlorhexidine, Fluoride, Lidocaine, Amphotericin, Miconazole, Nystatin, Fish oil, Ginkgo Biloba, Ginseng, Ginger, Purple coneflower, Saw Palmetto, Cetirizine, Levocetirizine, Loratadine, Diclofenac, Flurbiprofen, Acrivastine Pseudoephedrine, Loratadine Pseudoephedrine, Glucosamine, hyaluronic acid, Decapeptide KSL-W, Decapeptide KSL, Resveratrol, Misoprostol, Bupropion, Nicotine, Ondansetron HCl, Esomeprazole, Lansoprazole, Omeprazole, Pantoprazole, Rabeprazole, Bacteria and the like, Loperamide, Simethicone, Acetylsalicylic acid and others, Sucralfate, Vitamin A, Vitamin B1, Vitamin B12, Vitamin B2, Vitamin B6, Biotin, Vitamin C, Vitamin D, Vitamin E, Folinic acid, Vitamin K, Niacin, Q10, Clotrimazole, Fluconazole, Itraconazole, Ketoconazole, Terbinafine, Allopurinol, Probenecid, Atorvastatin, Fluvastatin, Lovastatin, Nicotinic acid, Pravastatin, Rosuvastatin, Simvastatin, Pilocarpine, Naproxen, Alendronate, Etidronate, Raloxifene, Risedronate, Benzodiazepines, Disulfiram, Naltrexone, Buprenorphine, Codeine, Dextropropoxyphene, Fentanyl, Hydromorphone, Ketobemidone, Ketoprofen, Methadone, Morphine, Naproxen, Nicomorphine, Oxycodone, Pethidine, Tramadol, Amoxicillin, Ampicillin, Azithromycin, Ciprofloxacin, Clarithromycin, Doxycyclin, Erythromycin, Fusidic acid, Lymecycline, Metronidazole, Moxifloxacin, Ofloxacin, Oxytetracycline, Phenoxymethylpenicillin, Rifamycins, Roxithromycin, Sulfamethizole, Tetracycline, Trimethoprim, Vancomycin, Acarbose, Glibenclamide, Gliclazide, Glimepiride, Glipizide, Insulin, Repaglinide, Tolbutamide, Oseltamivir, Aciclovir, Famciclovir, Penciclovir, Valganciclovir, Amlopidine, Diltiazem, Felodipine, Nifedipine, Verapamil, Finasteride, Minoxidil, Cocaine, Buphrenorphin, Clonidine, Methadone, Naltrexone, Calciumantagonists, Clonidine, Ergotamine, β-blockers, Aceclofenac, Celecoxib, Dexiprofen, Etodolac, Indometacin, Ketoprofen, Ketorolac, Lornoxicam, Meloxicam, Nabumetone, Oiroxicam, Parecoxib, Phenylbutazone, Piroxicam, Tiaprofenic acid, Tolfenamic acid, Aripiprazole, Chlorpromazine, Chlorprothixene, Clozapine, Flupentixol, Fluphenazine, Haloperidol, Lithium carbonate, Lithium citrate, Melperone, Penfluridol, Periciazine, Perphenazine, Pimozide, Pipamperone, Prochlorperazine, Risperidone, Thioridizin, Fluconazole, Itraconazole, Ketoconazole, Voriconazole, Opium, Benzodiazepines, Hydroxine, Meprobamate, Phenothiazine, Aluminiumaminoacetate, Esomeprazole, Famotidine, Magnesium oxide, Nizatide, Omeprazole, Pantoprazole, Fluconazole, Itraconazole, Ketoconazole, Metronidazole, Amphetamine, Atenolol, Bisoprolol fumarate, Metoprolol, Metropolol, Pindolol, Propranolol, Auranofin, and Bendazac.

Further examples of useful active ingredients include active ingredients selected from the therapeutical groups comprising: Analgesic, Anaestetic, Antipyretic, Anti allergic, Anti-arrytmic, Appetite suppressant, Antifungal, Anti-inflammatory, Broncho dilator, Cardiovascular drugs, Coronary dilator, Cerebral dilator, Peripheral vasodilator, Anti-infective, Psychotropic, Anti- manic, Stimulant, Antihistamine, Laxative, Decongestrant, Gastro-intestinal sedative, Sexual dysfunction agent, Desinfectants, Anti-diarrheal, Anti-anginal substance, Vasodilator, Anti-hypertensive agent, Vasoconstrictor, Migraine treating agent, Antibiotic, Tranquilizer, Ntipsychotic, Anti-tumor drug, Anticoagulant, Antithrombotic agent, Hypnotic, Sedative, Anti-emetic, Anti-, auseant, Anticonvulsant, Neuromuscular agent, Hyper and hypoglycaemic, Thyroid and antithyroid, Diuretic, Antispasmodic, Uterine relaxant, Anti-obesity agent, Anoretic, Spasnolytics, Anabolic agent, Erythropoietic agent, Anti-asthmatic, Expectorant, Cough suppressant, Mucolytic, Anti-uricemic agent, Dental vehicle, Breath freshener, Antacid, Anti-diuretc, Anti-flatulent, Betablokker, Teeth Whitener, Enzyme, Co-enzyme, Protein, Energy booster, Fiber, Probiotics, Prebiotics, Antimicrobial agent, NSAID, Anti-tussives, Decongestrants, Anti-histamines, Expectorants, Anti-diarrheals, Hydrogen antagonists, Proton pump inhibitors, General nonselective CNS depressants, General nonselective CNS stimulants, Selectively CNS function modyfying drugs, Antiparkinsonism, Narcotic-analgetics, Analgetic-antipyretics, Psychopharmacological drugs, and Sexual dysfunction agents.

Examples of useful active ingredients include: Casein glyco-macro-peptide (CGMP), Nicotine, Nicotine bitartrate, Nicotine sulphate, Nicotine tartrate, Nicotine pftalate, Nicotine lactate, Nicotinecitrate, Nicotine polacrilex, Triclosan, Cetyl pyridinium chloride, Domiphen bromide, Quarternary ammonium salts, Zinc components, Sanguinarine, Fluorides, Alexidine, Octonidine, EDTA, Aspirin, Acetaminophen, Ibuprofen, Ketoprofen, Diflunisal, Fenoprofen calcium, Naproxen, Tolmetin sodium, Indomethacin, Benzonatate, Caramiphen edisylate, Menthol, Dextromethorphan hydrobromide, Theobromine hydrochloride, Chlophendianol Hydrochloride, Pseudoephedrine Hydrochloride, Phenylephrine, Phenylpropanolamine, Pseudoephedrine sulphate, Brompheniramine maleate, Chlorpheniramine- maleate, Carbinoxamine maleate, Clemastine fumarate, Dexchlorpheniramine maleate, Dephenhydramine hydrochloride, Diphenpyralide hydrochloride, Azatadine maleate, Diphenhydramine citrate, Doxylamine succinate, Promethazine hydrochloride, Pyrilamine maleate, Tripellenamine citrate, Triprolidine hydrochloride, Acrivastine, Loratadine, Brompheniramine, Dexbrompheniamine, Guaifenesin, Ipecac, Potassium iodide, Terpin hydrate, Loperamide, Famotidine, Ranitidine, Omeprazole, Lansoprazole, Aliphatic alcohols, Barbiturates, Caffeine, Nicotine, Strychnine, Picrotoxin, Pentyenetetrazol, Phenyhydantoin, Phenobarbital, Primidone, Carbamazapine, Etoxsuximide, Methsuximide, Phensuximide, Trimethadione, Diazepam, Benzodiazepines, Phenacemide, Pheneturide, Acetazolamide, Sulthiame, Bromide, Levodopa, Amantadine, Morphine, Heroin, Hydromorphone, Metopon, Oxymorphone, Levophanol, Codeine, Hydrocodone, Xycodone, Nalorphine, Naloxone, Naltrexone, Salicylates, Phenylbutazone, Indomethacin, Phenacetin, Chlorpromazine, Methotrimeprazine, Haloperidol, Clozapine, Reserpine, Imipramine, Tranylcypromine, Phenelzine, Lithium, Sildenafil citrate, Tadalafil, and Vardenafil CL.

Examples of useful active ingredients include active ingredients selected from the groups of ace-inhibitors, antianginal drugs, anti- arrhythmias, anti-asthmatics, anti-cholesterolemics, analgesics, anesthetics, anticonvulsants, anti-depressants, antidiabetic agents, anti-diarrhea preparations, antidotes, anti-histamines, anti-hypertensive drugs, anti-inflammatory agents, anti-lipid agents, anti- manics, antinauseants, anti-stroke agents, anti-thyroid preparations, anti-tumor drugs, anti- viral agents, acne drugs, alkaloids, amino acid preparations, anti-tussives, anti- uricemic drugs, anti- viral drugs, anabolic preparations, systemic and non-systemic anti-infective agents, anti-neoplasties, antiparkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies such as sildenafil citrate, which is currently marketed as Viagra™, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids such as bromocriptine or nicotine, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumor drugs, anti-coagulants, anti-thrombotic drugs, hypnotics, anti-emetics, antinauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

Examples of active ingredients contemplated for use in the present invention can include antacids, H2-antagonists, and analgesics. For example, antacid dosages can be prepared using the ingredients calcium carbonate alone or in combination with magnesium hydroxide, and/or aluminum hydroxide. Moreover, antacids can be used in combination with H2-antagonists.

Analgesics include opiates and opiate derivatives, such as Oxycontin™, ibuprofen, aspirin, acetaminophen, and combinations thereof that may optionally include caffeine.

Other drug active ingredients for use in embodiments can include anti-diarrheals such as Immodium™ AD, anti-histamines, anti-tussives, decongestants, vitamins, and breath fresheners. Also contemplated for use herein are anxiolytics such as Xanax™; anti-psychotics such as Clozaril™ and Haldol™; non-steroidal anti-inflammatories (NSAID's) such as ibuprofen, naproxen sodium, Voltaren™ and Lodine™, anti-histamines such as Claritin™, Hismanal™, Relafen™, and Tavist™; antiemetics such as Kytril™ and Cesamet™; bronchodilators such as Bentolin™, Proventil™; anti-depressants such as Prozac™, Zoloft™, and Paxil™; anti-migraines such as Imigra™, ACE-inhibitors such as Vasotec™, Capoten™ and Zestril™; anti-Alzheimer's agents, such as Nicergoline™; and CaH-antagonists such as Procardia™, Adalat™, and Calan™.

The popular H2-antagonists which are contemplated for use in the present invention include cimetidine, ranitidine hydrochloride, famotidine, nizatidien, ebrotidine, mifentidine, roxatidine, pisatidine and aceroxatidine.

Active antacid ingredients can include, but are not limited to, the following: aluminum hydroxide, dihydroxyaluminum aminoacetate, aminoacetic acid, aluminum phosphate, dihydroxyaluminum sodium carbonate, bicarbonate, bismuth aluminate, bismuth carbonate, bismuth subcarbonate, bismuth subgallate, bismuth subnitrate, bismuth subsilysilate, calcium carbonate, calcium phosphate, citrate ion (acid or salt), amino acetic acid, hydrate magnesium aluminate sulfate, magaldrate, magnesium alumino silicate, magnesium carbonate, magnesium glycinate, magnesium hydroxide, magnesium oxide, magnesium trisilicate, milk solids, aluminum mono-ordibasic calcium phosphate, tricalcium phosphate, potassium bicarbonate, sodium tartrate, sodium bicarbonate, magnesium aluminosilicates, tartaric acids and salts. A variety of nutritional supplements may also be used as active ingredients including virtually any vitamin or mineral. For example, vitamin A, vitamin C, vitamin D, vitamin E, vitamin K, vitamin B6, vitamin B12, thiamine, riboflavin, biotin, folic acid, niacin, pantothenic acid, sodium, potassium, calcium, magnesium, phosphorus, sulfur, chlorine, iron, copper, iodine, zinc, selenium, manganese, choline, chromium, molybdenum, fluorine, cobalt and combinations thereof, may be used. Examples of nutritional supplements that can be used as active ingredients are set forth in U.S. Patent Application Publication Nos. 2003/0157213 A1, 2003/0206993 and 2003/0099741 A1 which are incorporated in their entirety herein by reference for all purposes. Various herbals may also be used as active ingredients such as those with various medicinal or dietary supplement properties. Herbals are generally aromatic plants or plant parts and or extracts thereof that can be used medicinally or for flavoring. Suitable herbals can be used singly or in various mixtures. Commonly used herbs include Echinacea, Goldenseal, Calendula, Rosemary, Thyme, Kava Kava, Aloe, Blood Root, Grapefruit Seed Extract, Black Cohosh, Ginseng, Guarana, Cranberry, Ginko Biloba, St. John's Wort, Evening Primrose Oil, Yohimbe Bark, Green Tea, Ma Huang, Maca, Bilberry, Lutein, and combinations thereof.

Especially when hydrophilic, encapsulation of the active will result in a delay in the release of the predominant amount of the active during consumption of a compressible chewing gum that includes the encapsulated active (e.g., as part of a delivery system added as an ingredient to the compressible chewing gum), in some embodiments, the release profile of the ingredient (e.g., the active) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more components of an effervescing system are managed for a compressible gum. The effervescent system may include one or more edible acids and one or more edible alkaline materials. The edible acid(s) and the edible alkaline material(s) may react together to generate effervescence. In some embodiments, the alkaline material(s) may be selected from, but is not limited to, alkali metal carbonates, alkali metal bicarbonates, alkaline earth metal carbonates, alkaline earth metal bicarbonates, and combinations thereof. The edible acid(s) may be selected from, but is not limited to, citric acid, phosphoric acid, tartaric acid, malic acid, ascorbic acid, and combinations thereof. In some embodiments, an effervescing system may include one or more other ingredients such as, for example, carbon dioxide, oral care ingredients, flavorants, etc.

For examples of use of an effervescing system in a chewing gum, refer to U.S. Provisional Patent No. 60/618,222 filed October 13, 2004, and entitled "Effervescent Pressed Gum Tablet Compositions," the contents of which are incorporated herein by reference for all purposes. Other examples can be found in U.S. Patent No. 6,235,318, the contents of which are incorporated herein by reference for all purposes. Typically, encapsulation of the one or more ingredients in an effervescing system will result in a delay in the release of the predominant amount of the one or more ingredients during consumption of a compressible chewing gum that includes the encapsulated one or more ingredients (e.g., as part of a delivery system added as an ingredient to the compressible chewing gum composition). The release profile of the one or more ingredients can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more appetite suppressors are managed for a compressible gum. Appetite suppressors can be ingredients such as fiber and protein that function to depress the desire to consume food. Appetite suppressors can also include benzphetamine, diethylpropion, mazindol, phendimetrazine, phentermine, hoodia (P57), Olibra™, ephedra, caffeine and combinations thereof. Appetite suppressors are also known by the following trade names: Adipex™, Adipost™, Bontril™ PDM, Bontril™ Slow Release, Didrex™, Fastin™, Ionamin™, Mazanor™, Melfiat™, Obenix™, Phendiet™, Phendiet-105™, Phentercot™, Phentride™, Plegine™, Prelu-2™, Pro-Fast™, PT 105™, Sanorex™, Tenuate™, Sanorex™, Tenuate™, Tenuate Dospan™, Tepanil Ten-Tab™, Teramine™, and Zantryl™. These and other suitable appetite suppressors are further described in the following U.S. patents, all of which are incorporated in their entirety by reference hereto: U.S. 6,838,431 to Portman, U.S. 6,716,815 to Portman, U.S. 6,558,690 to Portman, U.S. 6,468,962 to Portman, U.S. 6,436,899 to Portman.

Typically, encapsulation of the appetite suppressor will result in a delay in the release of the predominant amount of the appetite suppressor during consumption of a compressible chewing gum that includes the encapsulated appetite suppressor (e.g., as part of a delivery system added as an ingredient to the compressible chewing gum). In some embodiments, the release profile of the ingredient (e.g., the appetite suppressor) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more breath fresheners are managed for a compressible gum. Breath fresheners can include essential oils as well as various aldehydes, alcohols, and similar materials. In some embodiments, essential oils can include oils of spearmint, peppermint, wintergreen, sassafras, chlorophyll, citral, geraniol, cardamom, clove, sage, carvacrol, eucalyptus, cardamom, magnolia bark extract, marjoram, cinnamon, lemon, lime, grapefruit, and orange. In some embodiments, aldehydes such as cinnamic aldehyde and salicylaldehyde can be used. Additionally, chemicals such as menthol, carvone, iso-garrigol, and anethole can function as breath fresheners. Of these, the most commonly employed are oils of peppermint, spearmint and chlorophyll.

In addition to essential oils and chemicals derived from them, in some embodiments, breath fresheners can include but are not limited to zinc citrate, zinc acetate, zinc fluoride, zinc ammonium sulfate, zinc bromide, zinc iodide, zinc chloride, zinc nitrate, zinc flurosilicate, zinc gluconate, zinc tartarate, zinc succinate, zinc formate, zinc chromate, zinc phenol sulfonate, zinc dithionate, zinc sulfate, siliver nitrate, zinc salicylate, zinc glycerophosphate, copper nitrate, chlorophyll, copper chlorophyll, chlorophyllin, hydrogenated cottonseed oil, chlorine dioxide, beta cyclodextrin, zeolite, silica-based materials, carbon-based materials, enzymes such as laccase, and combinations thereof. In some embodiments, the release profiles of probiotics can be managed for a compressible gum including, but not limited to lactic acid producing microorganisms such as Bacillus coagulans, Bacillus subtilis, Bacillus laterosporus, Bacillus laevolacticus, Sporolactobacillus inulinus, Lactobacillus acidophilus, Lactobacillus curvatus, Lactobacillus plantarum, Lactobacillus jenseni, Lactobacillus casei, Lactobacillus fermentum, Lactococcus lactis, Pedioccocus acidilacti, Pedioccocus pentosaceus, Pedioccocus urinae, Leuconostoc mesenteroides, Bacillus coagulans, Bacillus subtilis, Bacillus laterosporus, Bacillus laevolacticus, Sporolactobacillus inulinus and mixtures thereof. Breath fresheners are also known by the following trade names: Retsyn™, Actizol™, and Nutrazin™. Examples of malodor-controlling compositions are also included in U.S. Patent No. 5,300,305 to Stapler et al. and in U.S. Patent Application Publication Nos. 2003/0215417 and 2004/0081713 which are incorporated in their entirety herein by reference for all purposes.

Typically, encapsulation of the breath-freshening ingredient will result in a delay in the release of the predominant amount of the active during consumption of a compressible chewing gum that includes the encapsulated breath-freshening ingredient (e.g., as part of a delivery system added as an ingredient to the compressible chewing gum composition). In some embodiments, the release profile of the ingredient (e.g., the breath-freshening ingredient) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more dental care ingredients may be managed for a compressible gum. Such dental care ingredients (also known as oral care ingredients) may include but are not limited to tooth whiteners, stain removers, oral cleaning, bleaching agents, desensitizing agents, dental remineralization agents, antibacterial agents, anticaries agents, plaque acid buffering agents, surfactants and anticalculus agents. Non-limiting examples of such ingredients can include, hydrolytic agents including proteolytic enzymes, abrasives such as hydrated silica, calcium carbonate, sodium bicarbonate and alumina, other active stain-removing components such as surface-active agents, including, but not limited to anionic surfactants such as sodium stearate, sodium palminate, sulfated butyl oleate, sodium oleate, salts of fumaric acid, glycerol, hydroxylated lecithin, sodium lauryl sulfate and chelators such as polyphosphates, which are typically employed as tartar control ingredients. In some embodiments, dental care ingredients can also include tetrasodium pyrophosphate and sodium tri-polyphosphate, sodium bicarbonate, sodium acid pyrophosphate, sodium tripolyphosphate, xylitol, sodium hexametaphosphate. In some embodiments, peroxides such as carbamide peroxide, calcium peroxide, magnesium peroxide, sodium peroxide, hydrogen peroxide, and peroxydiphospate are included. In some embodiments, potassium nitrate and potassium citrate are included. Other examples can include casein glycomacropeptide, calcium casein peptone-calcium phosphate, casein phosphopeptides, casein phosphopeptide- amorphous calcium phosphate (CPP-ACP), and amorphous calcium phosphate. Still other examples can include papaine, krillase, pepsin, trypsin, lysozyme, dextranase, mutanase, glycoamylase, amylase, glucose oxidase, and combinations thereof. Further examples can include surfactants such as sodium stearate, sodium ricinoleate, and sodium lauryl sulfate surfactants for use in some embodiments to achieve increased prophylactic action and to render the dental care ingredients more cosmetically acceptable. Surfactants can preferably be detersive materials which impart to the composition detersive and foaming properties. Suitable examples of surfactants are water-soluble salts of higher fatty acid monoglyceride monosulfates, such as the sodium salt of the monosulfated monoglyceride of hydgrogenated coconut oil fatty acids, higher alkyl sulfates such as sodium lauryl sulfate, alkyl aryl sulfonates such as sodium dodecyl benzene sulfonate, higher alkyl sulfoacetates, sodium lauryl sulfoacetate, higher fatty acid esters of 1,2-dihydroxy propane sulfonate, and the substantially saturated higher aliphatic acyl amides of lower aliphatic amino carboxylic acid compounds, such as those having 12 to 16 carbons in the fatty acid, alkyl or acyl radicals, and the like. Examples of the last mentioned amides are N-lauroyl sarcosine, and the sodium, potassium, and ethanolamine salts of N-lauroyl, N-myristoyl, or N-palmitoyl sarcosine. In addition to surfactants, dental care ingredients can include antibacterial agents such as, but not limited.to, triclosan, chlorhexidine, zinc citrate, silver nitrate, copper, limonene, and cetyl pyridinium chloride. In some embodiments, additional anticaries agents can include fluoride ions or fluorine-providing components such as inorganic fluoride salts. In some embodiments, soluble alkali metal salts, for example, sodium fluoride, potassium fluoride, sodium fluorosilicate, ammonium fluorosilicate, sodium monofluorophosphate, as well as tin fluorides, such as stannous fluoride and stannous chloride can be included. In some embodiments, a fluorine-containing compound having a beneficial effect on the care and hygiene of the oral cavity, e.g., diminution of enamel solubility in acid and protection of the teeth against decay may also be included as an ingredient. Examples thereof include sodium fluoride, stannous fluoride, potassium fluoride, potassium stannous fluoride (SnF.sub.2 -KF), sodium hexafluorostannate, stannous chlorofluoride, sodium fluorozirconate, and sodium monofluorophosphate. In some embodiments, urea is included. Further examples are included in the following U.S. patents and U.S. published patent applications, the contents of all of which are incorporated in their entirety herein by reference for all purposes: U.S. Patent Nos. 5,227,154 to Reynolds, 5,378,131 to Greenberg, 6,846,500 to Luo et al, 6,733,818 to Luo et al., 6,696,044 to Luo et al., 6,685,916 to Holme et al., 6,485,739 to Luo et al., 6,479,071 to Holme et al., 6,471,945 to Luo et al., U.S. Patent Publication Nos. 20050025721. to Holme et al., 2005008732 to Gebreselassie et al., and 20040136928 to Holme et al.

Typically, encapsulation of the active will result in a delay in the release of the predominant amount of the active during consumption of a compressible chewing gum that includes the encapsulated active (e.g., as part of a delivery system added as an ingredient to the compressible chewing gum composition). In some embodiments, the release profile of the ingredient (e.g., the dental care active) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc. In some embodiments, the release profiles of one or more flavor potentiators can be managed for a compressible gum. Flavor potentiators can consist of materials that may intensify, supplement, modify or enhance the taste and/or aroma perception of an original material without introducing a characteristic taste and/or aroma perception of their own. In some embodiments, potentiators designed to intensify, supplement, modify, or enhance the perception of flavor, sweetness, tartness, umami, kokumi, saltiness and combinations thereof can be included. In some embodiments, sweetness may be potentiated by the inclusion of monoammonium glycyrrhizinate, licorice glycyrrhizinates, citrus aurantium, maltol, ethyl maltol, vanilla, vanillin, and combinations thereof. In some embodiments, sugar acids, sodium chloride, potassium chloride, sodium acid sulfate, and combinations thereof may be included for flavor potentiation. In other examples, glutamates such as monosodium glutamate (MSG), monopotassium glutamate, hydrolyzed vegetable protein, hydrolyzed animal protein, yeast extract, and combinations thereof are included. Further examples can include glutathione, and nucleotides such as inosine monophosphate (IMP), disodium inosinate, xanthosine monophosphate, guanylate monophosphate (GMP), and combinations thereof. For bitterness blocking or taste masking, ingredients that interact with bitterness receptors to suppress bitterness or off tastes may be included. In some embodiments, adenosine monophosphate (AMP) can be included for bitterness suppression. Bitterness modification can also be accomplished by using sweetness or flavors with complementary bitter notes such as chocolate. Further examples of flavor potentiator compositions that impart kokumi are also included in U.S. Patent No. 5,679,397 to Kuroda et al,.

Typically, encapsulation of a flavor potentiator will result in a delay in the release of the predominant amount of the flavor potentiator during consumption of a compressible chewing gum that includes the encapsulated flavor potentiator (e.g., as part of a delivery system added as an ingredient to the compressible chewing gum composition). In some embodiments, the release profile of the ingredient (e.g., the flavor potentiator) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more acids may be managed for a compressible gum. Acids can include, but are not limited to acetic acid, adipic acid, ascorbic acid, butyric acid, citric acid, formic acid, fumaric acid, glyconic acid, lactic acid, phosphoric acid, malic acid, oxalic acid, succinic acid, tartaric acid and combinations thereof.

Typically, encapsulation of a food acid will result in a delay in the release of the predominant amount of the active during consumption of a compressible chewing gum that includes the encapsulated food acid (e.g., as part of a delivery system added as an ingredient to the compressible chewing gum). In some embodiments, the release profile of the ingredient (e.g., the food acid) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more micronutrients can be managed for a compressible gum. Micronutrients can include materials that have an impact on the nutritional wellbeing of an organism even though the quantity required by the organism to have the desired effect is small relative to macronutrients such as protein, carbohydrate, and fat. Micronutrients can include, but are not limited to vitamins, minerals, enzymes, phytochemicals, antioxidants, and combinations thereof. In some embodiments, vitamins can include fat soluble vitamins such as vitamin A, vitamin D, vitamin E, and vitamin K and combinations thereof, in some embodiments, vitamins can include water soluble vitamins such as vitamin C (ascorbic acid), the B vitamins (thiamine or B1, riboflavoin or B2, niacin or B3, pyridoxine or B6, folic acid or B9, cyanocobalimin or B12, pantothenic acid, biotin), and combinations thereof.

In some embodiments, minerals can include but are not limited to sodium, magnesium, chromium, iodine, iron, manganese, calcium, copper, fluoride, potassium, phosphorous, molybdenum, selenium, zinc, and combinations thereof.

In some embodiments micronutrients can include but are not limited to L- carnitine, choline, coenzyme Q10, alpha-lipoic acid, omega-3 -fatty acids, pepsin, phytase, trypsin, lipases, proteases, cellulases, and combinations thereof.

Antioxidants can include materials that scavenge free radicals. In some embodiments, antioxidants can include but are not limited to ascorbic acid, citric acid, rosemary oil, vitamin A, vitamin E, vitamin E phosphate, tocopherols, di-alpha-tocopheryl phosphate, tocotrienols, alpha lipoic acid, dihydrolipoic acid, xanthophylls, beta cryptoxanthin, lycopene, lutein, zeaxanthin, astaxanthin, beta-carotene, carotenes, mixed carotenoids, polyphenols, flavonoids, and combinations thereof.

In some embodiments, phytochemicals can include but are not limited to cartotenoids, chlorophyll, chlorophyllin, fiber, flavanoids, anthocyanins, cyaniding, delphinidin, malvidin, pelargonidin, peonidin, petunidin, flavanols, catechin, epicatechin, epigallocatechin, epigallocatechingallate, theaflavins, thearubigins, proanthocyanins, flavonols, quercetin, kaempferol, myricetin, isorhamnetin, flavononeshesperetin, naringenin, eriodictyol, tangeretin, flavones, apigenin, luteolin, lignans, phytoestrogens, resveratrol, isoflavones, daidzein, genistein, glycitein, soy isoflavones, and combinations thereof.
Typically, encapsulation of the micronutrient will result in a delay in the release of the predominant amount of the active during consumption of a compressible chewing gum that includes the encapsulated micronutrient (e.g., as part of a delivery system added as an ingredient to the compressible chewing gum). In some embodiments, the release profile of the ingredient (e.g., the micronutrient) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more mouth moisteners can be managed for a compressible gum. Mouth moisteners can include, but are not limited to, saliva stimulators such as acids and salts and combinations thereof. In some embodiments, acids can include acetic acid, adipic acid, ascorbic acid, butyric acid, citric acid, formic acid, fumaric acid, glyconic acid, lactic acid, phosphoric acid, malic acid, oxalic acid, succinic acid, tartaric acid and combinations thereof. Mouth moisteners can also include hydrocolloid materials that hydrate and may adhere to oral surface to provide a sensation of mouth moistening. Hydrocolloid materials can include naturally occurring materials such as plant exudates, seed gums, and seaweed extracts or they can be chemically modified materials such as cellulose, starch, or natural gum derivatives. In some embodiments, hydrocolloid materials can include pectin, gum arabic, acacia gum, alginates, agar, carageenans, guar gum, xanthan gum, locust bean gum, gelatin, gellan gum, galactomannans, tragacanth gum, karaya gum, curdlan, konjac, chitosan, xyloglucan, beta glucan, furcellaran, gum ghatti, tamarin, bacterial gums, and combinations thereof. Additionally, in some embodiments, modified natural gums such as propylene glycol alginate, carboxymethyl locust bean gum, low methoxyl pectin, and their combinations can be included. In some embodiments, modified celluloses can be included such as microcrystalline cellulose, carboxymethlcellulose (CMC), methylcellulose (MC), hydroxypropylniethylcellulose (HPCM), and hydroxypropylcellulose (MPC), and combinations thereof. Similarly, humectants which can provide a perception of mouth hydration can be included. Such humectants can include, but are not limited to glycerol, sorbitol, polyethylene glycol, erythritol, and xylitol. Additionally, in some embodiments, fats can provide a perception of mouth moistening. Such fats can include medium chain triglycerides, vegetable oils, fish oils, mineral oils, and combinations thereof. Typically, encapsulation of a mouth moistening agent will result in a delay in the release of the predominant amount of the active during consumption of a compressible chewing gum that includes the encapsulated mouth moistening agent (e.g., as part of a delivery system added as an ingredient to the compressible chewing gum). In some embodiments, the release profile of the ingredient (e.g., the mouth moistening agent) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more ingredients that soothe the throat can be managed for a compressible gum. Throat soothing ingredients can include analgesics, anesthetics, demulcents, antiseptic, and combinations thereof. In some embodiments, analgesics/anesthetics can include menthol, phenol, hexylresorcinol, benzocaine, dyclonine hydrochloride, benzyl alcohol, salicyl alcohol, and combinations thereof. In some embodiments, demulcents can include but are not limited to slippery elm bark, pectin, gelatin, and combinations thereof. In some embodiments, antiseptic ingredients can include cetylpyridinium chloride, domiphen bromide, dequalinium chloride, and combinations thereof.

In some embodiments, antitussive ingredients such as chlophedianol hydrochloride, codeine, codeine phosphate, codeine sulfate, dextromethorphan, dextromethorphan hydrobromide, diphenhydramine citrate, and diphenhydramine hydrochloride, and combinations thereof can be included.

In some embodiments, throat soothing agents such as honey, propolis, aloe vera, glycerine, menthol and combinations thereof can be included. In still other embodiments, cough suppressants can be included. Such cough suppressants can fall into two groups: those that alter the texture or production of phlegm such as mucolytics and expectorants; and those that suppress the coughing reflex such as codeine (narcotic cough suppressants), antihistamines, dextromethorphan and isoproterenol (non-narcotic cough suppressants). In some embodiments, ingredients from either or both groups can be included.

In still other embodiments, antitussives can include, but are not limited to, the group consisting of codeine, dextromethorphan, dextrorphan, diphenhydramine, hydrocodone, noscapine, oxycodone, pentoxyverine and combinations thereof. In some embodiments, antihistamines can include, but are not limited to, acrivastine, azatadine, brompheniramine, chlo[phi]heniramine, clemastine, cyproheptadine, dexbrompheniramine, dimenhydrinate, diphenhydramine, doxylamine, hydroxyzine, meclizine, phenindamine, phenyltoloxamine, promethazine, pyrilamine, tripelennamine, triprolidine and combinations thereof. In some embodiments, non-sedating antihistamines can include, but are not limited to, astemizole, cetirizine, ebastine, fexofenadine, loratidine, terfenadine, and combinations thereof.

In some embodiments, expectorants can include, but are not limited to, ammonium chloride, guaifenesin, ipecac fluid extract, potassium iodide and combinations thereof. In some embodiments, mucolytics can include, but are not limited to, acetylcycsteine, ambroxol, bromhexine and combinations thereof. In some embodiments, analgesic, antipyretic and anti-inflammatory agents can include, but are not limited to, acetaminophen, aspirin, diclofenac, diflunisal, etodolac, fenoprofen, flurbiprofen, ibuprofen, ketoprofen, ketorolac, nabumetone, naproxen, piroxicam, caffeine and mixtures thereof. In some embodiments, local anesthetics can include, but are not limited to, lidocaine, benzocaine, phenol, dyclonine, benzonotate and mixtures thereof. In some embodiments nasal decongestants and ingredients that provide the perception of nasal clearing can be included. In some embodiments, nasal decongestants can include but are not limited to phenylpropanolamine, pseudoephedrine, ephedrine, phenylephrine, oxymetazoline, and combinations thereof. In some embodiments ingredients that provide a perception of nasal clearing can include but are not limited to menthol, camphor, borneol, ephedrine, eucalyptus oil, peppermint oil, methyl salicylate, bornyl acetate, lavender oil, wasabi extracts, horseradish extracts, and combinations thereof. In some embodiments, a perception of nasal clearing can be provided by odoriferous essential oils, extracts from woods, gums, flowers and other botanicals, resins, animal secretions, and synthetic aromatic materials.

Typically, encapsulation of a throat care agent will result in a delay in the release of the predominant amount of the active during consumption of a compressible chewing gum that includes the encapsulated throat care agent (e.g. as part of a delivery system added as an ingredient to the compressible chewing gum). In some embodiments, the release profile of the ingredient (e.g. the dental care active) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, one or more colors can be included. As classified by the United States Food, Drug, and Cosmetic Act (21 C.F.R. 73), colors can include exempt from certification colors (sometimes referred to as natural even though they can be synthetically manufactured) and certified colors (sometimes referred to as artificial), or combinations thereof. In some embodiments, exempt from certification or natural colors can include, but are not limited to annatto extract, (E 160b), bixin, norbixin, astaxanthin, dehydrated beets (beet powder), beetroot red/betanin (E 162), ultramarine blue, canthaxanthin (E161g), cryptoxanthin (E161c), rubixanthin (E161d), violanxanthin (E161e), rhodoxanthin (E161f), caramel (E150(a-d)), β-apo-8'-carotenal (E160e), β-carotene (E160a), alpha carotene, gamma carotene, ethyl ester of beta-apo-8 carotenal (E160f), fiavoxanthin (E161a), lutein (E161b), cochineal extract (E120); carmine (E132), carmoisine/azorubine (E122), sodium copper chlorophyllin (E141), chlorophyll (E140), toasted partially defatted cooked cottonseed flour, ferrous gluconate, ferrous lactate, grape color extract, grape skin extract (enocianina), anthocyanins (E163), haematococcus algae meal, synthetic iron oxide, iron oxides and hydroxides (E172), fruit juice, vegetable juice, dried algae meal, tagetes (Aztec marigold) meal and extract, carrot oil, corn endosperm oil, paprika, paprika oleoresin, phaffia yeast, riboflavin (E101), saffron, titanium dioxide, turmeric (E100), turmeric oleoresin, amaranth (E123), capsanthin/capsorbin (E160c), lycopene (E160d), and combinations thereof.

In some embodiments, certified colors can include, but are not limited to, FD&C blue #1, FD&C blue #2, FD&C green #3, FD&C red #3, FD&C red #40, FD&C yellow #5 and FD&C yellow #6, tartrazine (E102), quinoline yellow (E104), sunset yellow (E110), ponceau (E124), erythrosine (E127), patent blue V (E131), titanium dioxide (E171), aluminum (E173), silver (E174), gold (E175), pigment rubine/lithol rubine BK (E180), calcium carbonate (E170), carbon black (E153), black PN/brilliant black BN (E151), green S/acid brilliant green BS (E142), and combinations thereof. In some embodiments, certified colors can include FD&C aluminum lakes. These consist of the aluminum salts of FD&C dyes extended on an insoluble substrate of alumina hydrate. Additionally, in some embodiments, certified colors can be included as calcium salts. Typically, encapsulation of a color will result in a delay in the release of the predominant amount of the active during consumption of a compressible chewing gum that includes the encapsulated color (e.g., as part of a delivery system added as an ingredient to the compressible chewing gum). In some embodiments, the release profile of the ingredient (e.g., the color) can be managed by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, a delivery system or compressible chewing gum may include two or more ingredients for which managed release from the compressible chewing gum during consumption of the compressible chewing gum is desired. In some embodiments, the ingredients may be encapsulated or otherwise included separately in different delivery systems. Alternatively, in some embodiments the ingredients may be encapsulated or otherwise included in the same delivery system. As another possibility, one or more of the ingredients may be free (e.g. unencapsulated) while one or more other ingredients may be encapsulated. A compressible chewing gum may include a group of ingredients for which managed release of the group during consumption of the compressible chewing gum is desired. Groups of two or more ingredients for which managed release from a compressible chewing gum during consumption of the compressible chewing gum may be desired include, but are not limited to: color and flavor, multiple flavors, multiple colors, cooling agent and flavor, warming agent and flavor, cooling agent and warming agent, cooling agent and high-intensity sweetener, warming agent and high-intensity sweetener, multiple cooling agents (e.g., WS-3 and WS-23, WS-3 and menthyl succinate), menthol and one or more cooling agents, menthol and one or more warming agents, multiple warming agents, high-intensity sweetener(s) and tooth whitening active(s), high-intensity sweetener(s) and breath-freshening active(s), an ingredient with some bitterness and a bitterness suppressor for the ingredient, multiple high-intensity sweeteners (e.g., ace-k and aspartame), multiple tooth whitening actives (e.g., an abrasive ingredient and an antimicrobial ingredient, a peroxide and a nitrate, a warming agent and a polyol, a cooling agent and a polyol, multiple polyols, a warming agent and micronutrient, a cooling agent and a micronutrient, a warming agent and a mouth moistening agent, a cooling agent and a mouth moistening agent, a warming agent and a throat care agent, a cooling agent and a throat care agent, a warming agent and a food acid, a cooling agent and food acid, a warming agent and an emulsifier/surfactant, a cooling agent and an emulsifier/surfactant, a warming agent and a color, a cooling agent and a color, a warming agent and a flavor potentiator, a cooling agent and a flavor potentiator, a warming agent with sweetness potentiator, a cooling agent with a sweetness potentiator, a warming agent and an appetite suppressant, a cooling agent and an appetite suppressant, a high-intensity sweetener and a flavor, a cooling agent and a teeth-whitening agent, a warming agent and a teeth-whitening agent, a warming agent and breath-freshening agent, a cooling agent and a breath-freshening agent, a cooling agent and an effervescing system, a warming agent and an effervescing system, a warming agent and an antimicrobial agent, a cooling agent and an antimicrobial agent, multiple anticalcums ingredients, multiple remineralization ingredients, multiple surfactants, remineralization ingredients with demineralization ingredients, acidic ingredients with acid buffering ingredients, anticalculus ingredients with antibacterial ingredients, remineralization ingredients with anticalculus ingredients, anticalculus ingredients with remineralization ingredients with antibacterial ingredients, surfactant ingredients with anticalculus ingredients, surfactant ingredients with antibacterial ingredients, surfactant ingredients with remineralization ingredients, surfactants with anticalculus ingredients with antibacterial ingredients, multiple types of vitamins or minerals, multiple micronutrients, multiple acids, multiple antimicrobial ingredients, multiple breath-freshening ingredients, breath-freshening ingredients and antimicrobial ingredients, multiple appetite suppressors, acids and bases that react to effervesce, a bitter compound with a high-intensity sweetener, a cooling agent and an appetite suppressant, a warming agent and an appetite suppressant, a high-intensity sweetener and an appetite suppressant, a high-intensity sweetener with an acid, a probiotic ingredient and a prebiotic ingredient, a vitamin and a mineral, a metabolic enhancement ingredient with a macronutrient, a metabolic enhancement ingredient with a micronutrient, an enzyme with a substrate, a high-intensity sweetener with a sweetness potentiator, a cooling compound with a cooling potentiator, a flavor with a flavor potentiator, a warming compound with a warming potentiator, a flavor with salt, a high-intensity sweetener with salt, an acid with salt, a cooling compound with salt, a warming compound with salt, a flavor with a surfactant, an astringent compound with an ingredient to provide a sensation of hydration, etc. In some embodiments, the multiple ingredients may be part of the same delivery system or may be part of different delivery systems. Different delivery systems may use the same or different encapsulating materials.

Typically, encapsulation of the multiple ingredients will result in a delay in the release of the predominant amount of the multiple ingredients during consumption of a compressible chewing gum that includes the encapsulated multiple ingredients (e.g. as part of a delivery system added as an ingredient to the compressible chewing gum). This may be particularly helpful in situations wherein separate encapsulation of the ingredients may cause them to release with different release profiles. For example, different high-intensity sweeteners may have different release profiles because they have different water solubilities or differences in other characteristics. Encapsulating them together may cause them to release more simultaneously.

In some embodiments, the release profile of the multiple ingredients can be managed for a compressible gum by managing various characteristics of the multiple ingredients, the delivery system containing the multiple ingredients, and/or the compressible chewing gum containing the delivery system and/or how the delivery system is made in a manner as previously discussed above.

The active ingredients mentioned above are meant as examples of active ingredients which could be applicable in a chewing gum granule or compressed chewing gum, however, this list should not be considered as exhaustive.

Active ingredients to be applied in tablets according to embodiments of the invention may be applied as such or be included or bonded in different ways, such as being part of an inclusion complex e.g. as described in US 5,866,179. A resin-bonding of nicotine is described in e.g. WO 2006/000232. Further conventional methods of applying active ingredients may obviously be applied within the scope of the invention.

The active ingredients may advantageously be applied in a gum base-containing module or a tablet-module substantially free of gum base depending on the applied type of active ingredient. If the active ingredient is of the pharmaceutical type, such ingredient may very often advantageously be comprised in a tablet module substantially free of gum base whereas taste relevant active ingredients advantageously may be added to the gum base-containing module and very often to both types of modules. The taste relevant active ingredient may both be added as separate particles which are mixed and compressed with gum base-containing particles in one module and it may be incorporated into gum base-containing granules.

In the present context, the terms granule and particle are used interchangeable in the sense that a granule or particle for use in a compression process is regarded to be a relatively small object, which together with other granules or particles may be compressed into a stable chewing gum tablet. The granules or particles may be produced in several different ways. A gum base-containing granule of particle may typically be produced substantially into the desired shape by means of an extrusion process or alternatively be produced on the basis of a gum base-containing mass which is subsequently separated into particles of a smaller size.

According to the present invention the embodiments mentioned in the text above may be combined in any order and in any sequence. Accordingly, the embodiments in the text above should not be read independently of each other.

The following examples illustrate the present invention. The examples are nonlimiting and are meant to be examples of a particular way to carry out the invention.

### EXAMPLE 1

### Preparation of gum base

A gum base is prepared, which comprises the following ingredients.

| Ingredients | Percent by weight |
|---|---|
| Elastomer | 10 |
| Natural resin | 28 |
| Synthetic resin | 22 |
| Fat/wax/emulsifiers | 23 |
| Fillers | 17 |

It should be emphasized that several other gum base compositions may be applied within the scope of the invention.

The elastomer and filler are added to a mixing kettle provided with mixing means like e.g. horizontally placed Z-shaped arms. The kettle has been preheated for 15 minutes to a temperature of about 120°C. The rubber is broken into small pieces and softened with mechanical action in the kettle.

The resin is slowly added to the elastomer until the mixture becomes homogeneous. The remaining resin is then added to the kettle and mixed for 10-20 minutes.

The softening ingredients are added and mixed for 20-40 minutes until the whole mixture becomes homogeneous.

The mixture is then discharged into the pan and allowed to cool to room temperature from the discharged temperature of 120 °C.

### EXAMPLE 2 - premixture of one flavor and modified CaCO3 with 33% load

1 part by weight of cinnamon aldehyde is mixed with 2 parts by weight of natural CaCO3 modified to a surface area at apx- 40 m2/g and remain to be a non-stick material.

### EXAMPLE 3 - premixture of one flavor and modified CaCO3 with 50% load

1 part by weight of orange flavor is mixed with 1 parts by weight of natural CaCO3 modified to a surface area at apx- 40 m2/g to form a free-flowing material.

### EXAMPLE 4- premixture of 2 flavors, sucralose and modified CaCO3

1 part by weight of cinnamon aldehyde and ½ part of menthol is mixed with 2 parts by weight of natural CaCO3 modified to a surface area at apx- 40 m2/g to form a free-flowing material. 2 parts of sucralose is added to the mixture and mixed.

### EXAMPLE 5 - premixture of 2 flavors, nicotin bound to resin and modified CaCO3

1 part by weight of cinnamon aldehyde and ½ part of menthol is mixed with 2 parts by weight of natural CaCO3 modified to a surface area at apx- 40 m2/g to form a free-flowing material. The nicotine and resin needed for one trial is added to the mixture and mixed.

### EXAMPLE 6 - premixture of nicotine and modified CaCO3

1 part by weight of pure nicotin is mixed with 2 parts by weight of natural CaCO3 modified to a surface area at apx- 40 m2/g to form a free-flowing material.

### EXAMPLE 7

### Preparation of nicotine-containing chewing gum cores with flavor mixture

Chewing gum cores are prepared by use of the gum base in example 1 and according to a conventional mechanical mixing procedure during moderate use of heating as described below.

| | |
|---|---|
| Gum base | 57.4% |
| Filler | 16.9% |
| Nicotine Polacrilex | |
| Nicotine | 0.2% |
| Ion exchange resin | 0.8% |

| Buffer agents | |
|---|---|
| Sodiumhydrogencarbonate | 1.0% |
| Sodium carbonate | 2.0% |
| Sorbitol powder | 19.1% |
| Flavor mixture from example 2-4: | 0,7% |
| Liquid sweetener | 1.5% |
| Intense sweetener | 0.4% |

Gum base and filler are mixed in a mixing kettle provided with mixing means like e.g. horizontally placed Z-shaped arms. The kettle has been preheated to a temperature of up to approximately 50 °C.

When the content is homogenous the other ingredients are added according to a specified time schedule. Nicotine is added in the first half of the mixing process and can be added as pure nicotine, as a nicotine salt or bound to an ion exchange resin, e.g. Amberlite IRP 64.

The chewing gum cores may be formulated with 0.1 - 8 mg of nicotine per piece preferably 2 or 4 mg. The pieces evaluated above comprise 2 mg nicotine complex. The flavormixture from example 2-4 or the like is added in the second half of the mixing process and can be added in 1 or more steps. The stability of nicotine in the gum increased significant.

### EXAMPLE 8

### Preparation of nicotine-containing chewing gum cores with flavor and nicotine mixture

The nicotine - mixture from example 5-6 and flavor-mixture (from ex. 2-4) is added to the chewing gum cores preparation as described in example 7. The stability of nicotine in the gum increased significant and the nicotine release was controlled.

### EXAMPLE 9

### Coating with flavor mixture

To a standard coating solution flavor-mixture (from ex. 2-4) is added and the nicotine chewing gum from ex. 7-8 are coated. The stability of the flavor and the gum increased significant.

### EXAMPLE 9

### Impregnation with flavor mixture

Flavor-mixture (from ex. 2-4) is used to perform impregnation as described in PCT/DK2008/000196, and the nicotine chewing gum are then coated. The amount of flavor impregnated increased significant.

## Claims

1. A chewing gum comprising a particulate material for controlled release of active ingredients, the particulate material comprising a combination of one or more active ingredients, wherein said one or more active ingredients comprises nicotine, and an inorganic mineral filler, wherein the one or more active ingredient is reversibly absorbed into and/or adsorbed onto the inorganic mineral filler, wherein the BET specific surface area of the inorganic mineral filler is above 15 m²/g, the BET specific surface area measured in accordance with ISO 9277, and wherein said inorganic mineral filler
comprises a natural calcium carbonate and/or a precipitated calcium carbonate (PCC).

2. A chewing gum according to claim 1, wherein nicotine is selected from nicotine salts, preferably tartrate, hydrogen tartrate, hydrochloride, acetate or salicylate, the free base form of nicotine, a nicotine derivative, nicotine in any non-covalent binding, and mixtures thereof.

3. A chewing gum according to any of the preceding claims, wherein the average grain diameter of the inorganic mineral filler is between 50 and 0.1 microns.

4. A chewing gum according to any of the preceding claims, wherein the weight of the dried inorganic mineral filler is increased by at least 1 % by weight at a relative humidity of 95 % at 25 degree Celcius compared to a relative humidity of 0 %.

5. A chewing gum according to any of the preceding claims, wherein the powder flow of the particulate material is higher than the powder flow of particulate material with a BET specific surface area of inorganic mineral filler below 15 m²/g, the BET specific surface area measured in accordance with ISO 9277.

6. A chewing gum according to any of the preceding claims, wherein the natural calcium carbonate is selected from the group consisting of a marble, a calcite, a chalk and a carbonate containing dolomite.

7. A chewing gum according to any of the preceding claims, wherein the inorganic mineral filler is obtainable by pre-treatment with one or more medium-strong to strong sources of H₃O⁺ ions and/or pre-treatment with an inorganic salt, such as a magnesium sulphate in combination with aluminium sulphate and/or zinc sulphate, and pre-treatment with gaseous CO₂, and for precipitated calcium carbonate preferably at a CO₂ gas flow rate of below 30 litres per minute at standard temperature and pressure per kilogram calcium hydroxide during precipitation.

8. The chewing gum according to any of the preceding claims, wherein the one or more active ingredients further comprises a flavoring agent.

9. The chewing gum according to any of the preceding claims , wherein the particulate material is contained in the chewing gum core.

10. The chewing gum according to any of the preceding claims, wherein the particulate material is contained in a sub-layer between the chewing gum core and an outer coating and/or in an outer coating

11. A method of manufacture a chewing gum comprising the steps of:
i) preparing a combination of an active ingredient being nicotine and an inorganic mineral filler, wherein the active ingredient is reversibly absorbed into and/or adsorbed onto the inorganic mineral filler, obtaining a particulate material according to any of claims 1 to 8, wherein the particulate material comprises a combination of one or more active ingredients, wherein said one or more active ingredients comprises nicotine, and an inorganic mineral filler, wherein the BET specific surface area of the inorganic mineral filler is above 15 m²/g, the BET specific surface area measured in accordance with ISO 9277, and wherein said inorganic mineral filler comprises a natural calcium carbonate and/or a precipitated calcium carbonate (PCC),
ii) formulating the particulate material with chewing gum ingredients, including gum base, obtaining a chewing gum having controlled release of the active ingredient, and
iii) optionally, pre-treating the inorganic mineral filler prior to step i) with one or more medium-strong to strong providers of H₃O⁺ ions and/or pre-treated with an inorganic salt, such as a magnesium sulphate in combination with aluminium sulphate and/or zinc sulphate, and pre-treated with gaseous CO₂, and for precipitated calcium carbonate preferably at a CO₂ gas flow rate of below 30 litres per minute at standard temperature and pressure per kilogram calcium hydroxide during precipitation.

12. A method of manufacture a chewing gum according to any of the claims 1-11.

## Patentansprüche

1. Kaugummi, umfassend ein teilchenförmiges Material für eine kontrollierte Freisetzung von Wirkstoffen, wobei das teilchenförmige Material eine Kombination von einem oder mehreren Wirkstoffen, wobei der eine oder die mehreren Wirkstoffe Nicotin umfasst, und einem anorganischen mineralischen Füllstoff umfasst, wobei der eine oder die mehreren Wirkstoffe reversibel in dem anorganischen mineralischen Füllstoff absorbiert und/oder an diesem adsorbiert ist, wobei die spezifische BET-Oberfläche des anorganischen mineralischen Füllstoffs über 15 m²/g beträgt, wobei die spezifische BET-Oberfläche gemäß ISO 9277 gemessen wird, und wobei der anorganische mineralische Füllstoff ein natürliches Calciumcarbonat und/oder ein präzipitiertes oder gefälltes Calciumcarbonat (PCC) umfasst.

2. Kaugummi nach Anspruch 1, wobei Nicotin aus Nicotinsalzen, vorzugsweise Tartrat, Hydrogentartrat, Hydrochlorid, Acetat oder Salicylat, der freien Basenform von Nicotin, einem Nicotinderivat, Nicotin in einer jeglichen nicht-kovalenten Bindung und Gemischen davon ausgewählt ist.

3. Kaugummi nach einem der vorangegangenen Ansprüche, wobei der durchschnittliche Korndurchmesser des anorganischen mineralischen Füllstoffs zwischen 50 und 0,1 Mikrometern liegt.

4. Kaugummi nach einem der vorangegangenen Ansprüche, wobei das Gewicht des getrockneten anorganischen mineralischen Füllstoffs bei einer relativen Feuchte von 95% bei 25°C verglichen mit einer relativen Feuchte von 0% um mindestens 1 Gew.-% erhöht ist.

5. Kaugummi nach einem der vorangegangenen Ansprüche, wobei der Pulverfluss des teilchenförmigen Materials höher ist als der Pulverfluss von teilchenförmigem Material mit einer spezifischen BET-Oberfläche von anorganischem mineralischem Füllstoff von unter 15 m²/g, wobei die spezifische BET-Oberfläche gemäß ISO 9277 gemessen wird.

6. Kaugummi nach einem der vorangegangenen Ansprüche, wobei das natürliche Calciumcarbonat aus der Gruppe bestehend aus einem Marmor, einem Calcit, einer Kreide und einem Carbonat enthaltenden Dolomit ausgewählt ist.

7. Kaugummi nach einem der vorangegangenen Ansprüche, wobei der anorganische mineralische Füllstoff erhältlich ist durch Vorbehandlung mit einer oder mehreren mittelstarken bis starken Quellen von H₃O⁺-Ionen und/oder Vorbehandlung mit einem anorganischen Salz, wie einem Magnesiumsulfat in Kombination mit Aluminiumsulfat und/oder Zinksulfat, und Vorbehandlung mit gasförmigem CO₂, und für präzipitiertes Calciumcarbonat vorzugsweise bei einer CO₂-Gasflussrate von unter 30 Litern pro Minute bei Standardtemperatur und -druck pro Kilogramm Calciumhydroxid während der Präzipitation.

8. Kaugummi nach einem der vorangegangenen Ansprüche, wobei der eine oder die mehreren Wirkstoffe des Weiteren einen Geschmack- oder Aromastoff umfasst..

9. Kaugummi nach einem der vorangegangenen Ansprüche, wobei das teilchenförmige Material in dem Kaugummikern enthalten ist.

10. Kaugummi nach einem der vorangegangenen Ansprüche, wobei das teilchenförmige Material in einer Unterschicht zwischen dem Kaugummikern und einem äußeren Überzug und/oder in einem äußeren Überzug enthalten ist.

11. Verfahren zur Herstellung eines Kaugummis, umfassend die folgenden Schritte:
i) Herstellen einer Kombination eines Wirkstoffs, welcher Nicotin ist, und eines anorganischen mineralischen Füllstoffs, wobei der Wirkstoff reversibel in dem anorganischen mineralischen Füllstoff absorbiert und/oder an diesem adsorbiert ist, wobei ein teilchenförmiges Material nach einem der Ansprüche 1 bis 8 erhalten wird, wobei das teilchenförmige Material eine Kombination von einem oder mehreren Wirkstoffen, wobei der eine oder die mehreren Wirkstoffe Nicotin umfasst, und einem anorganischen mineralischen Füllstoff umfasst, wobei die spezifische BET-Oberfläche des anorganischen mineralischen Füllstoffs über 15 m²/g beträgt, wobei die spezifische BET-Oberfläche gemäß ISO 9277 gemessen wird, und wobei der anorganische mineralische Füllstoff ein natürliches Calciumcarbonat und/oder ein präzipitiertes oder gefälltes Calciumcarbonat (PCC) umfasst.
ii) Formulieren des teilchenförmigen Materials mit Kaugummibestandteilen, einschließlich Gummigrundlage, wobei ein Kaugummi, welcher eine kontrollierte Freisetzung des Wirkstoffs aufweist, erhalten wird, und
iii) gegebenenfalls Vorbehandeln des anorganischen mineralischen Füllstoffs vor Schritt i) mit einer oder mehreren mittelstarken bis starken Quellen von H₃O⁺-Ionen und/oder vorbehandelt mit einem anorganischen Salz, wie einem Magnesiumsulfat in Kombination mit Aluminiumsulfat und/oder Zinksulfat, und vorbehandelt mit gasförmigem CO₂, und für präzipitiertes Calciumcarbonat vorzugsweise bei einer CO₂-Gasflussrate von unter 30 Litern pro Minute bei Standardtemperatur und -druck pro Kilogramm Calciumhydroxid während der Präzipitation.

12. Verfahren zur Herstellung eines Kaugummis nach einem der Ansprüche 1-11.

## Revendications

1. Gomme à mâcher comprenant un matériau particulaire pour la libération contrôlée de principes actifs, le matériau particulaire comprenant une combinaison d'un ou plusieurs principes actifs, dans laquelle lesdits un ou plusieurs principes actifs comprennent de la nicotine, et une charge minérale inorganique, dans laquelle les un ou plusieurs principes actifs sont absorbés de manière réversible dans et/ou adsorbés sur la charge minérale inorganique, dans laquelle la surface spécifique BET de la charge minérale inorganique est supérieure à 15 m²/g, la surface spécifique BET mesurée conformément à ISO 9277, et dans laquelle ladite charge minérale inorganique comprend un carbonate de calcium naturel et/ou un carbonate de calcium précipité (PCC).

2. Gomme à mâcher selon la revendication 1, dans laquelle la nicotine est sélectionnée parmi les sels de nicotine, de préférence le tartrate, le tartrate d'hydrogène, le chlorhydrate, l'acétate ou le salicylate, la forme base libre de la nicotine, un dérivé de nicotine, la nicotine dans toute liaison non covalente, et des mélanges de ceux-ci.

3. Gomme à mâcher selon l'une quelconque des revendications précédentes, dans laquelle le diamètre de grain moyen de la charge minérale inorganique est entre 50 et 0,1 micromètres.

4. Gomme à mâcher selon l'une quelconque des revendications précédentes, dans laquelle le poids de la charge minérale inorganique sèche est augmenté d'au moins 1 % en poids à une humidité relative de 95 % à 25 °Celsius par rapport à une humidité relative de 0 %.

5. Gomme à mâcher selon l'une quelconque des revendications précédentes, dans laquelle l'écoulement de poudre du matériau particulaire est supérieur à l'écoulement de poudre du matériau particulaire avec une surface spécifique BET de charge minérale inorganique inférieure à 15 m²/g, la surface spécifique BET mesurée conformément à ISO 9277.

6. Gomme à mâcher selon l'une quelconque des revendications précédentes, dans laquelle le carbonate de calcium naturel est sélectionné dans le groupe constitué d'un marbre, d'une calcite, d'une craie et d'une dolomite contenant du carbonate.

7. Gomme à mâcher selon l'une quelconque des revendications précédentes, dans laquelle la charge minérale inorganique peut être obtenue par prétraitement avec une ou plusieurs sources modérément fortes à fortes d'ions H₃O⁺ et/ou par prétraitement avec un sel inorganique, tel qu'un sulfate de magnésium en combinaison avec du sulfate d'aluminium et/ou du sulfate de zinc, et un prétraitement avec du CO₂ gazeux, et pour le carbonate de calcium précipité de préférence à un débit de gaz de CO₂ inférieur à 30 litres par minute à une température standard et une pression standard par kilogramme d'hydroxyde de calcium durant la précipitation.

8. Gomme à mâcher selon l'une quelconque des revendications précédentes, dans laquelle les un ou plusieurs principes actifs comprennent en outre un agent aromatisant.

9. Gomme à mâcher selon l'une quelconque des revendications précédentes, dans laquelle le matériau particulaire est contenu dans le coeur de gomme à mâcher.

10. Gomme à mâcher selon l'une quelconque des revendications précédentes, dans laquelle le matériau particulaire est contenu dans une sous-couche entre le coeur de gomme à mâcher et un revêtement extérieur et/ou dans un revêtement extérieur.

11. Procédé de fabrication d'une gomme à mâcher comprenant les étapes suivantes :
i) préparation d'une combinaison d'un principe actif étant de la nicotine et d'une charge minérale inorganique, dans lequel le principe actif est absorbé de manière réversible dans et/ou absorbé sur la charge minérale inorganique, pour obtenir un matériau particulaire selon l'une quelconque des revendications 1 à 8, dans lequel le matériau particulaire comprend une combinaison d'un ou plusieurs principes actifs, dans lequel lesdits un ou plusieurs principes actifs comprennent de la nicotine, et une charge minérale inorganique, dans lequel la surface spécifique BET de la charge minérale inorganique est supérieure à 15 m²/g, la surface spécifique BET mesurée conformément à ISO 9277, et dans lequel ladite charge minérale inorganique comprend un carbonate de calcium naturel et/ou un carbonate de calcium précipité (PCC),
ii) formulation du matériau particulaire avec des ingrédients de gomme à mâcher, incluant une base de gomme, pour obtenir une gomme à mâcher ayant une libération contrôlée du principe actif, et
iii) optionnellement, prétraitement de la charge minérale inorganique avant l'étape i) avec un ou plusieurs fournisseurs modérément forts à forts d'ions H₃O⁺ et/ou prétraitée avec un sel inorganique, tel qu'un sulfate de magnésium en combinaison avec du sulfate d'aluminium et/ou du sulfate de zinc, et prétraitée avec du CO₂ gazeux, et pour le carbonate de calcium précipité de préférence à un débit de gaz de CO₂ inférieur à 30 litres par minute à une température standard et une pression standard par kilogramme d'hydroxyde de calcium durant la précipitation.

12. Procédé de fabrication d'une gomme à mâcher selon l'une quelconque des revendications 1 à 11.
